# EUROPEAN PATENT SPECIFICATION

(11) **EP 3 497 114 B1**
(45) Date of publication and mention of the grant of the patent: **16.10.2024**
(21) Application number: 17838864.1
(22) Date of filing: 24.05.2017
(51) Int. Cl.: C12N 15/113, C12N 15/11

(54) **ANDROGEN RECEPTOR ANTISENSE OLIGONUCLEOTIDES**
ANDROGENREZEPTOR-ANTISENSE-OLIGONUKLEOTIDE
OLIGONUCLÉOTIDES ANTISENS DU RÉCEPTEUR DES ANDROGÈNES

(30) Priority: 08.08.2016 US 201662372035 P
(43) Date of publication of application: 19.06.2019
(73) Proprietor: Olipass Corporation, Yongin-si, Gyeonggi-do 17105 (KR)
(72) Inventor: CHUNG, Shin, Yongin-si Gyeonggi-do 16990 (KR); JUNG, Daram, Hwaseong-si Gyeonggi-do (KR); CHO, Bongjun, Yongin-si Gyeonggi-do 17077 (KR); JANG, Kangwon, Yongin-si Gyeonggi-do 16998 (KR); YOON, Heungsik, Seongnam-si Gyeonggi-do 13623 (KR)
(74) Representative: HGF
(86) International application number: PCT/IB2017/000697
(87) International publication number: WO 2018/029517

(56) References cited:
- WO-A1-2014/059364
- WO-A1-2018/122610
- WO-A2-2009/113828
- US-B1- 6 617 422
- YAMAMOTO, YOSHIAKI ET AL.: "Generation 2.5 antisense oligonucleotides targeting the androgen receptor and its splice variants suppress enzalutamide-resistant prostate cancer cell growth", CLINICAL CANCER RESEARCH, vol. 21, no. 7, 29 January 2015 (2015-01-29), pages 1675 - 1687, XP055463386
- DEHM, SCOTT M. ET AL.: "Splicing of a novel androgen receptor exon generates a constitutively active androgen receptor that mediates prostate cancer therapy resistance", CANCER RESEARCH, vol. 68, no. 13, 1 July 2008 (2008-07-01), pages 5469 - 5477, XP008154829
- KARRAS, JAMES G. ET AL.: "Peptide nucleic acids are potent modulators of endogenous pre-mRNA splicing of the murine interleukin-5 receptor- a chain", BIOCHEMISTRY, vol. 40, no. 26, 6 May 2001 (2001-05-06), pages 7853 - 7859, XP055463390

## Description

This invention relates to peptide nucleic acid derivatives targeting androgen receptor pre-mRNA for the treatment of dermatological indications or conditions mediated by androgenic activity.

### Background of Invention

Alopecia is a disorder characterized by hair loss and hair thinning initially on the scalp. Androgenic alopecia, also referred to as "male pattern baldness," is caused by overt androgenic activity in hair follicles and surrounding tissue.

While androgenic alopecia affects both men and women, the disorder often shows up differently in men versus women. Males are likely to experience spot baldness, and females are more likely to experience overall hair thinning on the scalp. The prevalence of androgenic alopecia in males aged 30 to 50 is approximately 58% [J. Invest. Dermatol. vol 9, 296-300 (1997)]. Androgenic alopecia is caused by changes in male steroid hormones known as androgens [New Engl. J. Med. vol 341, 491-7 (1999**);** Mol. Cell Endocrinol. vol 198, 89-95 (2002)].

Androgens regulate the release of sebum in sebaceous glands, the hair growth in hair follicles, libido systemically, and so on. Androgens stimulate the gradual transformation of small vellus follicles, making non-pigmented, fine, and short hairs in some areas to larger terminal follicles (e.g. face). In contrast to this androgen action on terminal follicles, however, gradual regression of terminal hair follicles to vellus follicles occurs on the temples and scalp vertex, which is often called as `androgen paradox'. [Expert Opin. Drug Discov. vol 10, 269-292 (2015)]

Androgenic Alopecia and DHT: 5α-reductase reduces testosterone into 5 α - dihydrotestosterone (DHT), an androgen more potent and effective than testosterone. A substantial increase in DHT production in frontal anagen hair follicles was observed in young balding males compared with non-balding males. [(Ind. J. Dermatol. Vene. Leprol. vol 79, 613-625 (2013)] Males with androgenic alopecia tend to show a lower level of "total testosterone" than those without androgenic alopecia. Instead, the DHT level is higher in males with androgenic alopecia than in those without androgenic alopecia. DHT is produced from testosterone by 5α-reductase. Males with androgenic alopecia express a higher level of 5 a -reductase in hair follicles than those without androgenic alopecia. DHT is highly responsible for miniaturization of hair follicles, and therefore androgenic alopecia. [Endocrinology, vol 151, 2373-2380 (2010)]

Finasteride and dutasteride inhibit 5α-reductase, and therefore decrease the DHT level available to androgen receptors in hair follicles and surrounding tissue. The two small molecule inhibitors have been used to treat male pattern baldness despite adverse effects originating from the down-regulation of systemic androgenic activity. The adverse effects include sexual dysfunction, dizziness, weakness, headache, runny nose, skin rash, and so on. [New Engl. J. Med. vol 362, 1237-8 (2010)].

Topical AR Antagonist: Androgens express their pharmacologic activities by binding to androgen receptor (AR). AR antagonists bind to AR and inhibit the physiological function of androgens, and therefore may be used to treat androgenic alopecia if properly delivered to hair follicles and surrounding tissue. In order to avoid side effects incurred by the inhibition of systemic androgenic activity, AR antagonists are topically administered directly to scalp tissue.

Ketoconazole possesses weak AR antagonistic activity in addition to its famous antifungal activity. A shampoo containing 2% ketoconazole (under a commercial brand name of Nizoral^{®}) has been used to topically treat androgenic hair loss. [J. Dermatol. Sci. vol 45(1), 66-68 (2007)]

Topilutamide is an AR antagonist known as fludiril. Topilutamide is marketed as a 2% topical formulation to treat androgenic alopecia in a number of European countries with a brand name of "Eucapil". [Dermatol. Surg. vol 28(8), 678-685 (2002)]

AR Protein or mRNA in Hair Follicles: In male and female subjects with androgenic alopecia, AR expression was found to be higher in frontal hair follicles than in occipital hair follicles. [J. Investig. Dermatol. vol 109, 296-300 (1997)] If AR expression is down-regulated by an agent selectively in hair follicles and surrounding tissue, such agent may safely treat androgenic alopecia without incurring adverse events caused by the systemic down-regulation of androgenic activity. In another literature, females with androgenic alopecia were found to show a higher level of AR mRNA in frontal and parietal hair follicles than in occipital hair follicles. [Genetics Mol. Res. vol 12(2), 1834-1840 (2013)]

Ribosomal Protein Synthesis: Proteins are encoded by DNA (2-deoxyribose nucleic acid). In response to cellular stimulation, DNA is transcribed to produce pre-mRNA (pre-messenger ribonucleic acid) in the nucleus. The introns of pre-mRNA are enzymatically spliced out to yield mRNA (messenger ribonucleic acid), which is then translocated into the cytosolic compartment. In the cytosol, a complex of translational machinery called ribosome binds to mRNA and carries out the protein synthesis as it scans the genetic information encoded along the mRNA. [Biochemistry vol 41, 4503-4510 (2002); Cancer Res. vol 48, 2659-2668 (1988)]

An oligonucleotide binding to RNA in a sequence specific manner (i.e. complementarily) is called antisense oligonucleotide (ASO). ASO may tightly bind to an mRNA and inhibit the protein synthesis by ribosome along the mRNA in the cytosol. ASO needs to be present within cell in order to inhibit the ribosomal protein synthesis of its target protein.

Splicing Process: DNA is transcribed to produce pre-mRNA (pre-messenger ribonucleic acid) in the nucleus. Pre-mRNA is then processed into mRNA following deletion of introns by a series of complex reactions collectively called "splicing" as schematically summarized in the diagram below. [Ann. Rev. Biochem. 72(1), 291-336 (2003); Nature Rev. Mol. Cell Biol. 6(5), 386-398 (2005); Nature Rev. Mol. Cell Biol. 15(2), 108-121 (2014)]

Splicing is initiated by forming "splicesome E complex" (i.e. early splicesome complex) between pre-mRNA and splicing adapter factors. In "splicesome E complex", U1 binds to the junction of exon N and intron N, and U2AF³⁵ binds to the junction of intron N and exon (N+1). Thus the junctions of exon/intron or intron/exon are critical to the formation of the early splicesome complex. "Splicesome E complex" evolves into "splicesome A complex" following additional complexation with U2. The "splicesome A complex" undergoes a series of complex reactions to delete or splice out the intron to adjoin the neighboring exons.

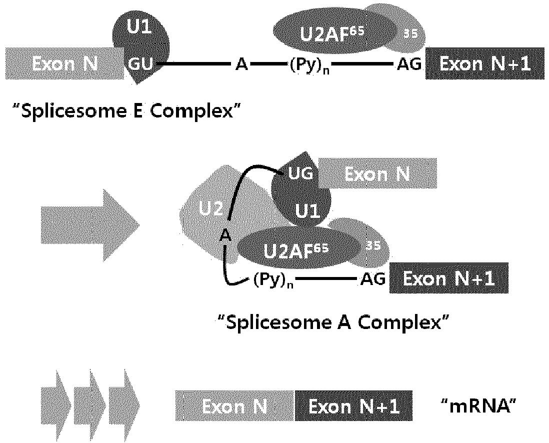
U1
Exon N GU A (Py)n AG Exon N+1
U2AF65 35
"Splicesome E Complex"
UG Exon N
U2
U1
A
U2AF65 35
(Py)n AG Exon N+1
"Splicesome A Complex"
Exon N Exon N+1 "mRNA"

Antisense Inhibition of Splicing: In the nucleus, ASO may tightly bind to a certain position within a pre-mRNA, and can interfere with the splicing process of the pre-mRNA into mRNA, producing the full-length mRNA or mRNA variant(s) lacking the target exon. Such mRNA(s) is called "splice variant(s)", encodes protein(s) smaller than the protein encoded by the full-length mRNA.

In principle, splicing can be interrupted by inhibiting the formation of "splicesome E complex". If an ASO tightly binds to a junction of (5' → 3') exon-intron, i.e. "5' splice site", the ASO blocks the complex formation between the pre-mRNA and factor U1, and therefore the formation of "splicesome E complex". Likewise, "splicesome E complex" cannot be formed if an ASO tightly binds to a junction of (5' → 3') intron-exon, i.e. "3' splice site".

Unnatural Oligonucleotides: DNA or RNA oligonucleotide is susceptible to degradation by endogenous nucleases, limiting their therapeutic utility. To date, a large number of unnatural oligonucleotides have been developed and studied intensively. [Clin. Exp. Pharmacol. Physiol. vol 33, 533-540 (2006)] Some of them were found to show extended metabolic stability compared to DNA and RNA. Provided below are the chemical structures for a few number of representative unnatural oligonucleotides. Such oligonucleotide predictably binds to its complementary nucleic acid as DNA or RNA does.

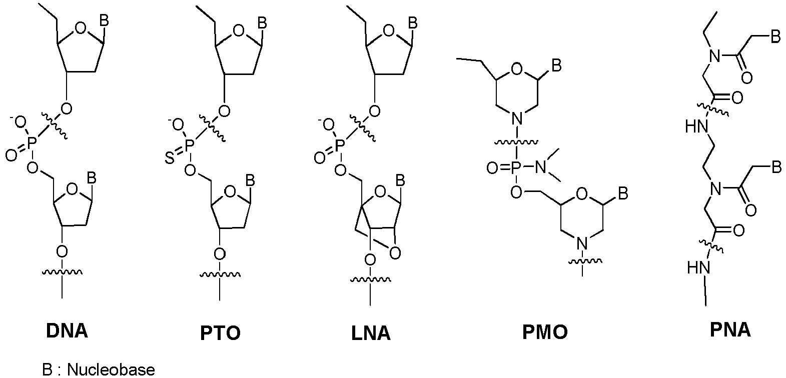

Phosphorothioate Oligonucleotide: Phosphorothioate oligonucleotide (PTO) is a DNA analog with one of the backbone phosphate oxygen atoms replaced with sulfur atom per monomer. Such a small structural change made PTO comparatively resistant to degradation by nucleases. [Ann. Rev. Biochem. vol 54, 367-402 (1985)]

Reflecting the structural similarity of backbone between PTO and DNA, they both poorly penetrate cell membrane in most mammalian cell types. For some types of cells abundantly expressing transporter(s) for DNA, however, DNA and PTO show good cellular uptake. Systemically administered PTOs are known to readily distribute to the liver and kidney. [Nucleic Acids Res. vol 25, 3290-3296 (1997)]

In order to increase PTO's in vitro cell membrane permeability, lipofection has been widely practiced. However, lipofection physically alters cell membrane, causes cytotoxicity, and therefore would not be safe for long term therapeutic use.

Over the past 30 years, antisense PTOs and variants of PTOs have been clinically evaluated to treat cancers, immunological disorders, metabolic diseases, and so on. [Biochemistry vol 41, 4503-4510 (2002); Clin. Exp. Pharmacol. Physiol. vol 33, 533-540 (2006)] Many of such antisense drug candidates have not been successfully developed partly due to PTO's poor cell membrane permeability. In order to overcome the poor membrane permeability, PTO needs to be administered at high dose for therapeutic activity. However, PTOs are known to be associated with dose-limiting toxicity including increased coagulation time, complement activation, tubular nephropathy, Kupffer cell activation, and immune stimulation including splenomegaly, lymphoid hyperplasia, mononuclear cell infiltration. [Clin. Exp. Pharmacol. Physiol. vol 33, 533-540 (2006)]

Many antisense PTOs have been found to show due clinical activity for diseases with a significant contribution from the liver or kidney. Mipomersen is a PTO analog which inhibits the synthesis of apoB-100, a protein involved in LDL cholesterol transport. Mipomersen manifested due clinical activity in a certain population of atherosclerosis patients most likely due to its preferential distribution to the liver. [Circulation vol 118(7), 743-753 (2008)] ISIS-113715 is a PTO antisense analog inhibiting the synthesis of protein tyrosine phosphatase 1B (PTP1B), and was found to show therapeutic activity in type II diabetes patients. [Curr. Opin. Mol. Ther. vol 6, 331-336 (2004)]

Locked Nucleic Acid: In locked nucleic acid (LNA), the backbone ribose ring of RNA is structurally constrained to increase the binding affinity for RNA or DNA. Thus, LNA may be regarded as a high affinity DNA or RNA analog. [Biochemistry vol 45, 7347-7355 (2006)] Like PTO, LNA also shows poor cell membrane permeability.

Phosphorodiamidate Morpholino Oligonucleotide: In phosphorodiamidate morpholino oligonucleotide (PMO), the backbone phosphate and 2-deoxyribose of DNA are replaced with phosphoamidite and morpholine, respectively. [Appl. Microbiol. Biotechnol. vol 71, 575-586 (2006)] Whilst the DNA backbone is negatively charged, the PMO backbone is not charged. Thus the binding between PMO and mRNA is free of electrostatic repulsion between the backbones, and tends to be stronger than that between DNA and mRNA. Since PMO is structurally very different from DNA, PMO wouldn't be recognized by the hepatic transporter(s) recognizing DNA or RNA. However, PMO doesn't readily penetrate cell membrane, either.

Peptide Nucleic Acid: Peptide nucleic acid (PNA) is a polypeptide with N-(2-aminoethyl)glycine as the unit backbone, and was discovered by Dr. Nielsen and colleagues. [Science vol 254, 1497-1500 (1991)] The chemical structure and abbreviated nomenclature of the prototype PNA are illustrated with the drawing provided below. Like DNA and RNA, PNA also selectively binds to complementary nucleic acid. [Nature (London) vol 365, 566-568 (1992)] In binding to complementary nucleic acid, the N-terminus of PNA is regarded as equivalent to the "5'-end" of DNA or RNA, and the C-terminus of PNA as equivalent to the "3'-end" of DNA or RNA.

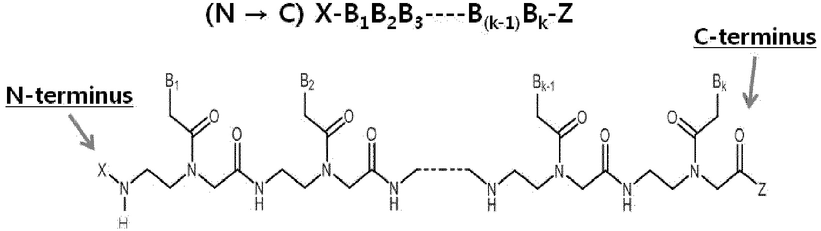

Like PMO, the PNA backbone is not charged. Thus the binding between PNA and RNA tends to be stronger than that between DNA and RNA. Since PNA is markedly different from DNA in the chemical structure, PNA wouldn't be recognized by the hepatic transporter(s) recognizing DNA, and would show a tissue distribution profile different from that of DNA or PTO. However, PNA also poorly penetrates mammalian cell membrane. (Adv. Drug Delivery Rev. vol 55, 267-280, 2003)

Modified Nucleobases to Improve Membrane Permeability of PNA: PNA was made highly permeable to mammalian cell membrane by introducing modified nucleobases with a cationic lipid or its equivalent covalently attached thereto. The chemical structures of such modified nucleobases are provided above. Such modified nucleobases of cytosine, adenine, and guanine were found to predictably and complementarily hybridize with guanine, thymine, and cytosine, respectively. [PCT Appl. No. PCT/KR2009/001256 (published as WO 2009/113828); EP2268607; US8680253]

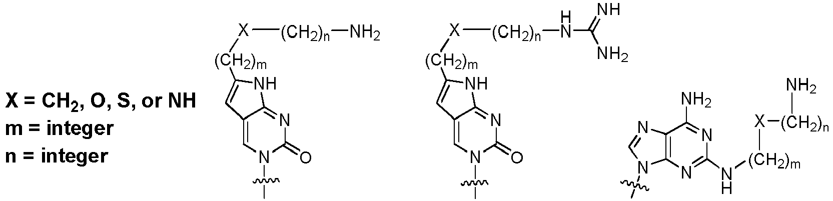

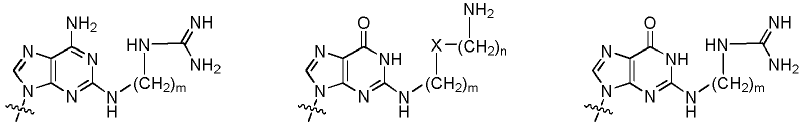

Incorporation of such modified nucleobases onto PNA resembles situations of lipofection. By lipofection, oligonucleotide molecules are wrapped with cationic lipid molecules such as lipofectamine, and such lipofectamine/oligonucleotide complexes tend to penetrate cell membrane rather easily as compared to naked oligonucleotide molecules.

In addition to good membrane permeability, those PNA derivatives were found to possess ultra-strong affinity for complementary nucleic acid. For example, introduction of 4 to 5 modified nucleobases onto 11- to 13-mer PNA derivatives easily yielded a Tₘ gain of 20°C or higher in duplex formation with complementary DNA. Such PNA derivatives are highly sensitive to a single base mismatch. A single base mismatch resulted in a Tₘ loss of 11 to 22°C depending on the type of modified base as well as PNA sequence.

AR Antisense Oligonucleotide (AR ASO): In principle, an ASO targeting the AR mRNA can inhibit ribosomal protein synthesis of androgen receptor. There are reported cases of AR ASOs inhibiting AR expression in cells. For example, EZN-4176, an LNA/DNA gapmer complementarily targeting the AR mRNA, down-regulated AR expression in tumor cells as well as in tumors of animal models for prostate cancer. [Mol. Cancer. Ther. vol 10(12), 2309-2319 (2011)].

ASOs targeting either exon 1 or exon 8 of the AR mRNA inhibited AR expression in prostate cancer cells as well as in tumors of animal models for prostate cancer resistant to chemotherapy with Enzalutamide, an AR antagonist. [Clin. Cancer Res. vol 21(7), 1675-1687 (2015)]

AR Down-regulation in Hair Follicles by Topical Application of AR ASO: Down-regulation of androgenic activity in hair follicles and surrounding tissue may be achieved by inhibiting AR expression in hair follicles and surrounding tissue. AR expression in hair follicles can be down-regulated with an AR ASO, if the ASO is delivered into hair follicles and surrounding tissue.

In order to avoid the side effects from the down-regulation of systemic androgenic activity, it is desired to have AR expression down-regulated locally in hair follicles and surrounding tissue for the treatment of androgenic alopecia. Topical application of an AR ASO to scalp skin would be the safest mode to inhibit AR expression locally in hair follicles and surrounding tissue, if the ASO is made or formulated to be readily delivered into hair follicles. To date, AR ASOs have been hardly used for topical treatment of androgenic alopecia. AR ASOs have been evaluated mostly for systemic administration to treat prostate cancer resistant to androgen ablation therapy.

### Brief Description of Drawings

**Figures 1(A)****-(C).** Examples of natural or unnatural (modified) nucleobases selectable for the peptide nucleic acid derivative of **Formula I.**
**Figures 2(A)****-(E).** Examples of substituents selectable for the peptide nucleic acid derivative of **Formula I.**
**Figure 3****.** Chemical structures of PNA monomers with natural or modified nucleobase.
**Figure 4****.** Chemical structures for abbreviations of N- or C-terminus substituents.
**Figure 5(A)****.** Chemical structure for the PNA derivative of "(N —> C) Fethoc-GA(5)A-GC(102)C-A(5)GG-C(102)AA(5)-G-NH₂".
**Figure 5(B)****.** Chemical structure for the PNA derivative of "(N —> C) Benzoyl-Lys-Val-C(102)TT-A(5)CC-A(5)GG-C(102)AA(5)-G-NH₂".
**Figure 6****.** Chemical structures for Fmoc-PNA monomers employed to synthesize the PNA derivatives of this invention.
**Figures 7(A)****-(B).** C₁₈-reverse phase HPLC chromatograms of "ASO 1" before and after HPLC purification, respectively.
**Figure 8(A)****.** Electrophoretic analysis of the nested PCR products of MCF7 cells treated with 0 aM (negative control), 3 aM, 30 aM, 300 aM, or 3 fM "ASO 5".
**Figure 8(B)****.** Schematic representation of the PCR band for the skipping of exons 4-5 along with sequencing data.
**Figure 9(A)****.** Changes in the relative levels of exons 4-6 in MCF7 cells treated with "ASO 5" at 0 zM (negative control), or 1 zM to 1 aM for 5 hours. (statistical analysis by student's t-test)
**Figure 9(B)****.** Changes in the relative levels of exons 4-6 in MCF7 cells treated with "ASO 1" at 0 zM (negative control), or 1 zM to 1 aM for 5 hours. (statistical analysis by student's t-test)
**Figure 9(C)****.** Changes in the relative levels of exons 4-6 in MCF7 cells treated with "ASO 10" at 0 zM (negative control), or 1 zM to 1 aM for 5 hours. (statistical analysis by student's t-test)
**Figure 10(A)****.** Western blot data for MCF7 cells treated with "ASO 1" at 0 zM (negative control), or 100 zM to 300 aM.
**Figure 10(B)****.** Western blot data for MCF7 cells treated with "ASO 5" at 0 zM (negative control), or 10 zM to 30 aM.
**Figure 11(A)****.** Hair growth images by group in the skin area of hair removal with days after hair removal.
**Figure 11(B)****.** Relative brightness scores of the "ASO 1" treatment groups against the negative control (vehicle) group. (statistical analysis by student's t-test)
**Figure 12****.** Representative sets of AR IHC (red) and DAPI (blue) fluorescence images for skin samples obtained from animals treated with "ASO 1" at 0 fM (negative control, vehicle), 0.2 fM or 1 fM.
**Figure 13****.** Relative brightness scores of the "ASO 5" treatment groups against the negative control (vehicle) group. (statistical analysis by student's t-test)
**Figure 14(A)****.** Relative brightness scores of the "ASO 10" treatment groups against the negative control (vehicle) group. (statistical analysis by student's t-test)
**Figure 14(B)****.** Representative sets of AR IHC (red) and DAPI (blue) fluorescence images for skin samples obtained from animals treated with "ASO 10" at 0 (negative control, vehicle), 1, 5 or 25 fM.
**Figure 15****.** qPCR data for the relative AR mRNA level by TaqMan assay in MCF7 cells treated with "ASO 10" at 0 (negative control), 1 , 10, 100, or 1,000 zM for 24 hours against the negative control. (statistical analysis by student's t-test)
**Figure 16****.** Representative sets of AR IHC (red) and DAPI (blue) fluorescence images for tissue samples obtained from mice subcutaneously administered with "ASO 10" at 0 (negative control), 0.01 or 0.1 pmole/Kg, 2X per week for 4 weeks.

### Summary of Invention

The present invention provides a peptide nucleic acid derivative represented by **Formula I,** or a pharmaceutically acceptable salt thereof: 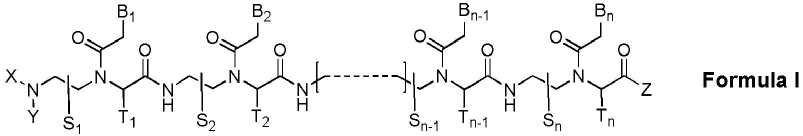 wherein,
n is an integer between 11 and 21;
the compound of **Formula I** possesses at least a 9-mer complementary overlap with a 17-mer RNA sequence of [(5' ----+ 3') CCUUGCCUGGUAAGGAA] within the human androgen receptor pre-mRNA;
S₁, S₂, ..., Sₙ₋₁, Sₙ, T₁, T₂, ..., Tₙ₋₁, and Tₙ independently represent deuterido, hydrido, substituted or non-substituted alkyl, or substituted or non-substituted aryl radical;
X and Y independently represent hydrido [H], formyl [H-C(=O)-], aminocarbonyl [NH₂-C(=O)-], substituted or non-substituted alkyl, substituted or non-substituted aryl, substituted or non-substituted alkylacyl, substituted or non-substituted arylacyl, substituted or non-substituted alkyloxycarbonyl, substituted or non-substituted aryloxycarbonyl, substituted or non-substituted alkylaminocarbonyl, substituted or non-substituted arylaminocarbonyl, substituted or non-substituted alkylsulfonyl, or substituted or non-substituted arylsulfonyl radical;
Z represents hydrido, hydroxy, substituted or non-substituted alkyloxy, substituted or non-substituted aryloxy, substituted or non-substituted amino, substituted or non-substituted alkyl, or substituted or non-substituted aryl radical;
B₁, B₂, ..., Bₙ₋₁, and Bₙ are independently selected from natural nucleobases including adenine, thymine, guanine, cytosine and uracil, and unnatural nucleobases; and
at least four of B₁, B₂, ..., Bₙ₋₁, and Bₙ are independently selected from unnatural nucleobases with a substituted or non-substituted amino radical covalently linked to the nucleobase moiety.

The compound of **Formula I** induces alternative splicing of the human AR pre-mRNA, yields AR mRNA splice variant(s) lacking "exon 5", and therefore is useful to safely treat dermatological indications or conditions involving androgenic activity upon topical administration.

### Description of Invention

The present invention provides a peptide nucleic acid derivative represented by **Formula I,** or a pharmaceutically acceptable salt thereof: 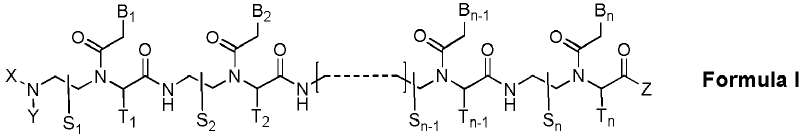 wherein,
n is an integer between 11 and 21;
the compound of **Formula I** possesses at least a 9-mer complementary overlap with a 17-mer RNA sequence of [(5' ----+ 3') CCUUGCCUGGUAAGGAA] within the human androgen receptor pre-mRNA;
S₁, S₂, ..., Sₙ₋₁, Sₙ, T₁, T₂, ..., Tₙ₋₁, and Tₙ independently represent deuterido, hydrido, substituted or non-substituted alkyl, or substituted or non-substituted aryl radical;
X and Y independently represent hydrido [H], formyl [H-C(=O)-], aminocarbonyl [NH₂-C(=O)-], substituted or non-substituted alkyl, substituted or non-substituted aryl, substituted or non-substituted alkylacyl, substituted or non-substituted arylacyl, substituted or non-substituted alkyloxycarbonyl, substituted or non-substituted aryloxycarbonyl, substituted or non-substituted alkylaminocarbonyl, substituted or non-substituted arylaminocarbonyl, substituted or non-substituted alkylsulfonyl, or substituted or non-substituted arylsulfonyl radical;
Z represents hydrido, hydroxy, substituted or non-substituted alkyloxy, substituted or non-substituted aryloxy, substituted or non-substituted amino, substituted or non-substituted alkyl, or substituted or non-substituted aryl radical;
B₁, B₂, ..., Bₙ₋₁, and Bₙ are independently selected from natural nucleobases including adenine, thymine, guanine, cytosine and uracil, and unnatural nucleobases; and
at least four of B₁, B₂, ..., Bₙ₋₁, and Bₙ are independently selected from unnatural nucleobases with a substituted or non-substituted amino radical covalently linked to the nucleobase moiety.

The compound of **Formula I** induces alternative splicing of the human AR pre-mRNA, yields AR mRNA splice variant(s) lacking "exon 5", and therefore is useful to safely treat dermatological indications or conditions involving androgenic activity upon topical administration.

The condition that "n is an integer between 11 and 21" literally states that n is an integer selectable from a group of integers of 12, 13, 14, 15, 16, 17, 18, 19, and 20.

The compound of **Formula I** tightly binds to the 5' splice site of "exon 5" of the human AR pre-mRNA transcribed from the human AR gene. [NCBI Reference Sequence: NC_000023.11] The 40-mer AR pre-mRNA sequence consisting of a 20-mer from "exon 5" and a 20-mer from "intron 5" unequivocally reads [(5' —> 3') GUGGGCCAAGGCCUUGCCUG-GUAAGGAAAAGGGAAGUGGG], although the exon and intron number may vary depending on AR mRNA transcript. The 40-mer pre-mRNA sequence may be alternatively denoted as [(5' ----+ 3') GUGGGCCAAGGCCUUGCCUG | guaaggaaaagggaaguggg], wherein the exon and intron sequences are expressed with "capital" and "small" letters, respectively, and the exon/intron junction is expressed with " | ".

The 17-mer pre-mRNA sequence of [(5' —> 3') CCUUGCCUGGUAAGGAA] adopted to describe the compound of **Formula I** in this invention consists of 9-mer in the AR "exon 5" and 8-mer in the AR "intron 5". Thus the 17-mer pre-mRNA sequence may alternatively read [(5' —> 3') CCUUGCCUG | guaaggaa].

The compound of **Formula I** tightly binds to the target 5' splice site of exon 5 in the human AR pre-mRNA, and interferes with the formation of "splicesome early complex" involving the compound's target exon. Since the compound of this invention sterically inhibits the formation of "splicesome early complex", the AR "exon 5" is spliced out to yield an AR mRNA splice variant or variants lacking "exon 5". Consequently the compound of this invention induces the skipping of "exon 5".

The compound of **Formula I** tightly binds to the complementary DNA as exemplified in the prior art [PCT/KR2009/001256, published as WO 2009/113828]. The duplex between the PNA derivative of **Formula I** and its full-length complementary DNA or RNA shows a Tₘ value too high to be reliably determined in aqueous buffer. The PNA compound of **Formula I** still yields high Tₘ values with complementary DNAs of shorter length, for example, 10-mer.

Owing to the high binding affinity, the PNA derivative of this invention potently induces the skipping of "exon 5" in cells even with a complementary overlap of as small as 9-mer with the 5' splice site of "exon 5", although such a small number of overlap may increase the risk of cross reactivity with other pre-mRNAs. If the PNA derivative of this invention is used for topical therapeutic purposes, the risk of the cross reactivity is predicted to be considerably attenuated.

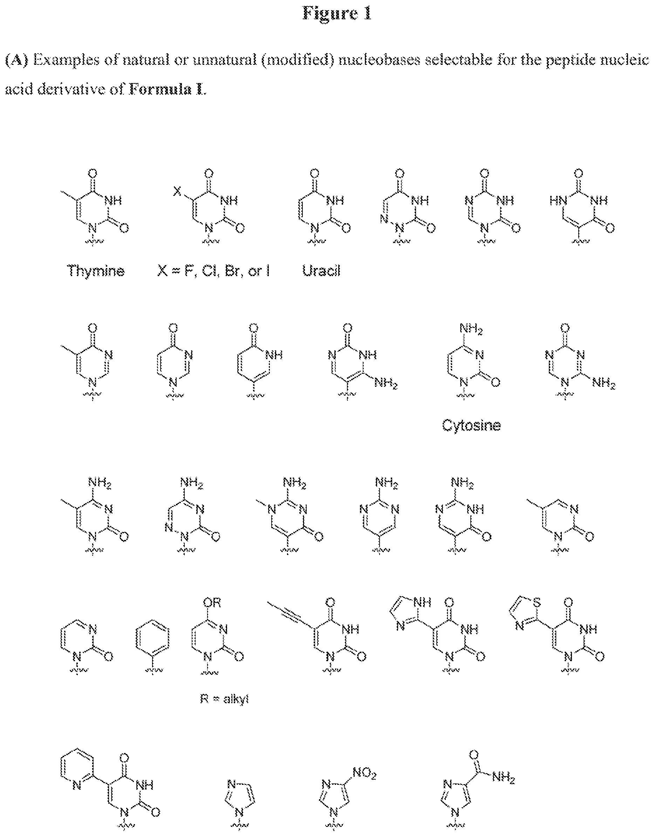

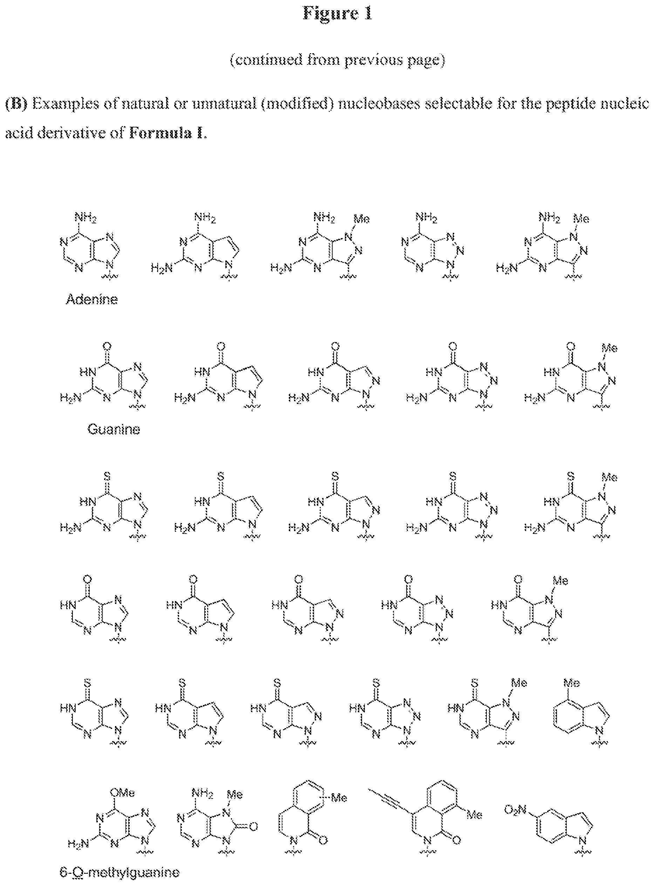

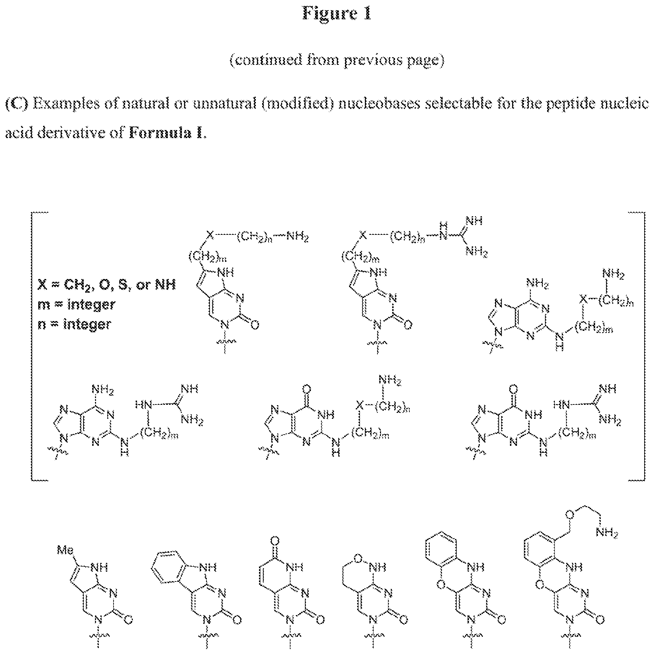

The chemical structures of natural or unnatural nucleobases in the PNA derivative of **Formula I** are exemplified in Figures 1(A)-(C). Natural (i.e. naturally occurring) or unnatural (i.e. non-naturally occurring) nucleobases of this invention comprise but are not limited to the nucleobases provided in Figures 1(A)-(C). Provision of such unnatural nucleobases is to illustrate the diversity of allowable nucleobases, and therefore should not be interpreted to limit the scope of the present invention. A skilled person in the field may easily figure out that variations of unnatural nucleobases are possible for specific positions in the PNA compound of **Formula I** as long as such variations meet the desired complementarity with its target pre-mRNA sequence.

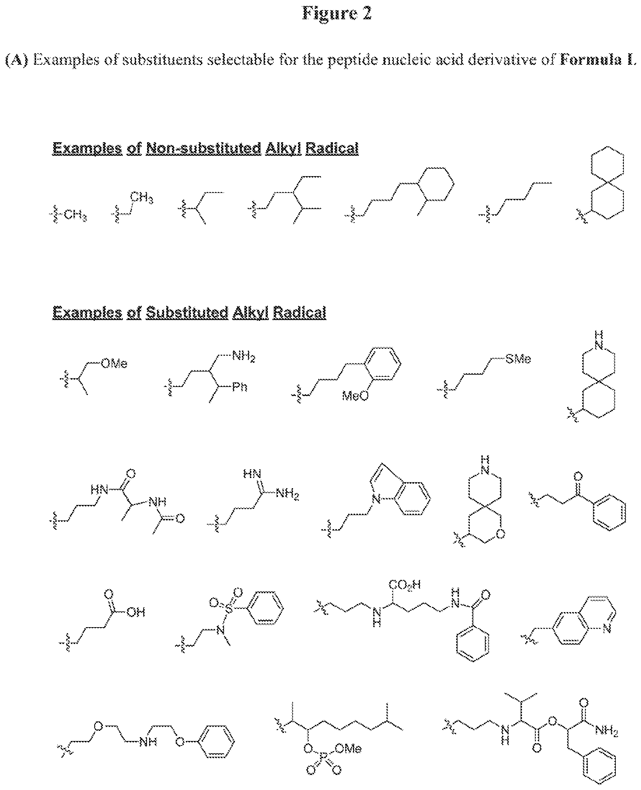

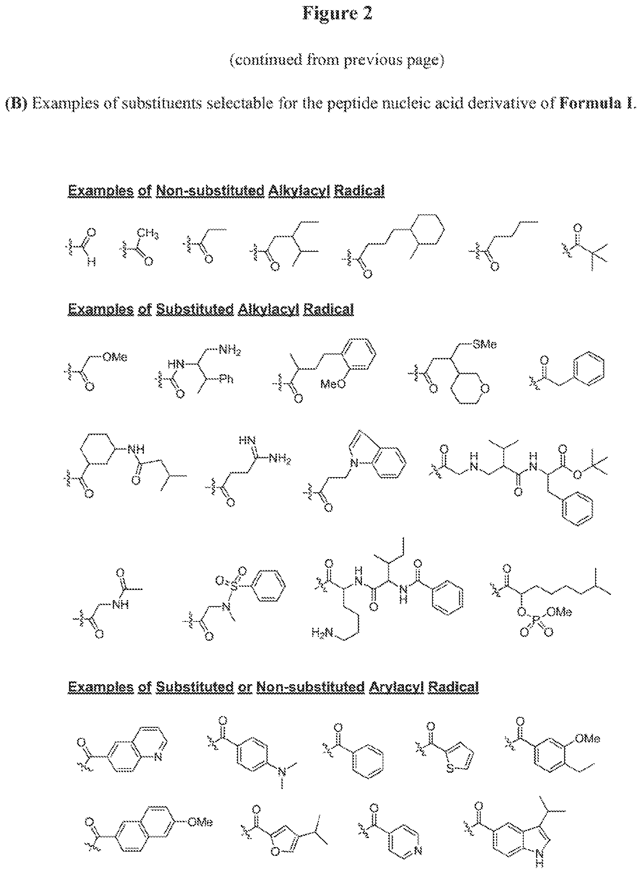

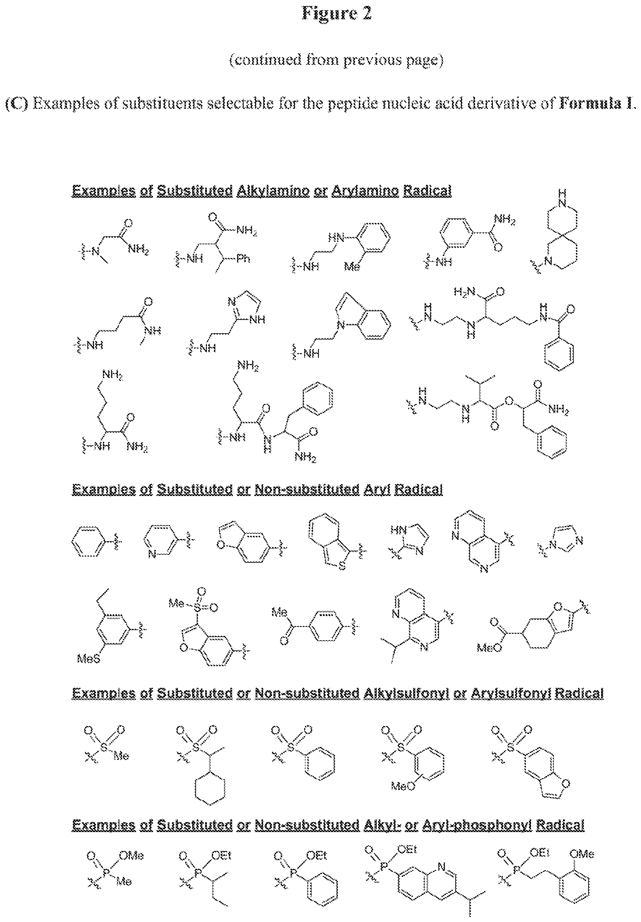

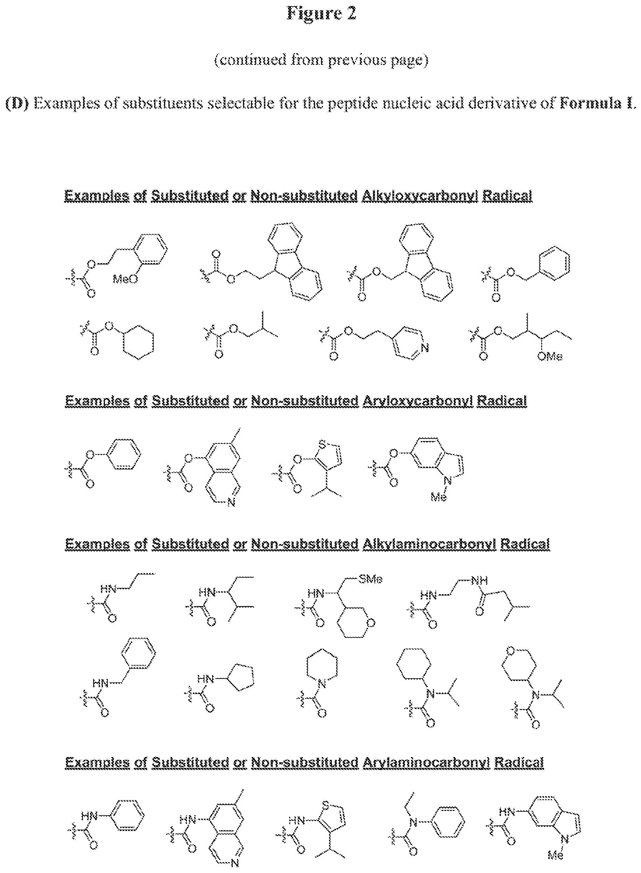

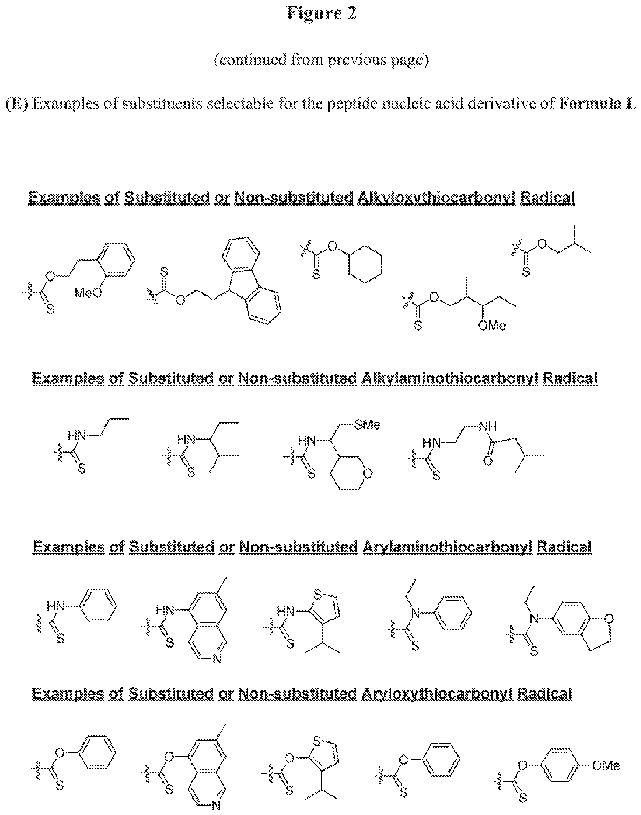

The substituents adopted to describe the PNA derivative of **Formula I** are exemplified in Figures 2(A)-(E). Figure 2(A) provides examples for substituted or non-substituted alkyl radicals. Substituted or non-substituted alkylacyl and substituted or non-substituted alkylacyl aryl acyl radicals are exemplified in Figure 2(B). Figure 2(C) illustrates examples for substituted or non-substituted alkylamino, substituted or non-substituted arylamino, substituted or non-substituted aryl, substituted or non-substituted alkylsulfonyl or arylsulfonyl, and substituted or non-substituted alkylphosphonyl or arylphosphonyl radicals. Figure 2(D) provides examples for substituted or non-substituted alkyloxycarbonyl or aryloxycarbonyl, substituted or non-substituted alkyl aminocarbonyl or arylaminocarbonyl radicals. In Figure 2(E) are provided examples for substituted or non-substituted alkylaminothiocarbonyl, substituted or non-substituted arylaminothiocarbonyl, substituted or non-substituted alkyloxythiocarbonyl, and substituted or non-substituted aryloxythiocarbonyl radicals. Provision of such exemplary substituents is to illustrate the diversity of allowable substituents, and therefore should not be interpreted to limit the scope of the present invention. A skilled person in the field may easily figure out that oligonucleotide sequence is the overriding factor for sequence specific binding of an oligonucleotide to the target pre-mRNA sequence over substituents in the N-terminus or C-terminus.

The compound of **Formula I** possesses good cell permeability and can be readily delivered into cell if treated as "naked" oligonucleotide as exemplified in the prior art [PCT/KR2009/001256, published as WO 2009/113828]. Thus the compound of this invention induces the skipping of "exon 5" in the human AR pre-mRNA to yield AR mRNA splice variant(s) lacking AR "exon 5" in cells treated with the compound of **Formula I** as "naked" oligonucleotide. The compound of **Formula I** does not require any means or formulations for delivery into cell to potently induce the skipping of the target exon in cells. The compound of **Formula I** readily induces the skipping of the AR "exon 5" in cells treated with the compound of this invention as "naked" oligonucleotide at sub-femtomolar concentration.

Owing to the good cell or membrane permeability, the PNA derivative of **Formula I** can be topically administered as "naked" oligonucleotide to induce the skipping of the AR "exon 5" in target skin. The compound of **Formula I** does not require a formulation to increase trans-dermal delivery for a topical therapeutic or biological activity. Usually the compound of **Formula I** is dissolved in water and co-solvent, and topically or trans-dermally administered at sub-picomolar concentration to elicit the desired therapeutic or biological activity in the target skin. The compound of this invention does not need to be heavily or invasively formulated to elicit the topical therapeutic activity.

The compound of **Formula I** may be used as combined with a pharmaceutically acceptable acid or base including but not limited to sodium hydroxide, potassium hydroxide, hydrochloric acid, methanesulfonic acid, citric acid, trifluoroacetic acid, and so on.

The PNA derivative of **Formula I** or a pharmaceutically acceptable salt thereof can be administered to a subject in combination with a pharmaceutically acceptable adjuvant including but not limited to citric acid, hydrochloric acid, tartaric acid, stearic acid, polyethyleneglycol, polypropyleneglycol, ethanol, isopropanol, sodium bicarbonate, distilled water, preservative(s), and so on.

The compound of the present invention can be topically administered to a subject at a therapeutically or biologically effective concentration ranging from 1 aM to higher than 1 nM, which would vary depending on the dosing schedule, conditions or situations of the subject, and so on.

Of highest interest is a PNA derivative of **Formula I,** or a pharmaceutically acceptable salt thereof:
wherein,
n is an integer between 11 and 15;
the compound of **Formula I** possesses at least a 11-mer complementary overlap with the 17-mer RNA sequence of [(5' → 3') CCUUGCCUGGUAAGGAA] within the human AR pre-mRNA;
the compound of **Formula I** is fully complementary to a pre-mRNA sequence within the human AR pre-mRNA;
S₁, S₂, ..., Sₙ₋₁, Sₙ, T₁, T₂, ..., Tₙ₋₁, and Tₙ are hydrido radical;
X is hydrido radical;
Y represents substituted or non-substituted alkylacyl, or substituted or non-substituted alkyloxycarbonyl radical;
Z represents substituted or non-substituted amino radical;
B₁, B₂, ..., Bₙ₋₁, and Bₙ are independently selected from adenine, thymine, guanine, cytosine, and unnatural nucleobases;
at least five of B₁, B₂, ..., Bₙ₋₁, and Bₙ are independently selected from unnatural nucleobases represented by **Formula II, Formula III,** or **Formula IV;** 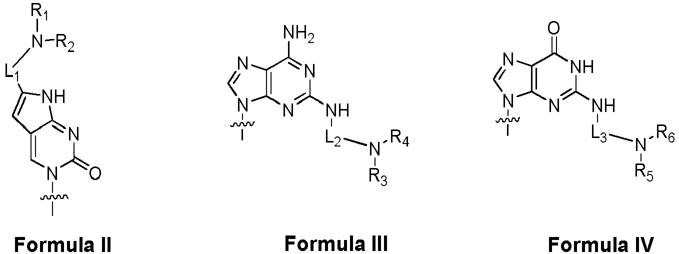 wherein
   R₁, R₂, R₃, R₄, R₅, and R₆ are hydrido radical;
   L₁ represents -(CH₂)₂-O-(CH₂)₂-, -CH₂-O-(CH₂)₂-, -CH₂-O-(CH₂)₃-, -CH₂-O-(CH₂)₄-, or -CH₂-O-(CH₂)₅- with the right end being directly linked to the basic amino group; and,
   L₂ and L₃ are independently selected from -(CH₂)₂-O-(CH₂)₂-, -(CH₂)₃-O-(CH₂)₂-, - (CH₂)₂-O-(CH₂)₃-, -(CH₂)₂-, -(CH₂)₃-, -(CH₂)₄-, -(CH₂)₅-, -(CH₂)₆-, -(CH₂)₇-, and -(CH₂)₈-with the right end being directly linked to the basic amino group.

Of specific interest is a PNA derivative of **Formula I** which is selected from the group of compounds provided below, or a pharmaceutically acceptable salt thereof:
(N → C) Fmoc-GA(5)A-GC(1O2)C-A(5)GG-C(1O2)AA(5)-G-NH₂;
(N → C) Fethoc-GA(5)A-GC(1O2)C-A(5)GG-C(1O2)AA(5)-G-NH₂;
(N → C) Ac-GA(5)A-GC(1O2)C-A(5)GG-C(1O2)AA(5)-G-NH₂;
(N → C) Benzoyl-GA(5)A-GC(1O2)C-A(2O2)GG-C(1O2)AA(5)-G-NH₂;
(N → C) Piv-GA(5)A-GC(1O2)C-A(5)GG-C(1O2)AA(5)-G-NH₂;
(N ----+ C) Methyl-GA(5)A-GC(1O2)C-A(5)GG-C(1O2)AA(5)-G-NH₂;
(N ----+ C) n-Propyl-GA(5)A-GC(1O2)C-A(5)GG-C(1O2)AA(5)-G-NH₂;
(N ----+ C) Fmoc-Lys-GA(5)A-GC(1O2)C-A(5)GG-C(1O2)AA(5)-G-NH₂;
(N ----+ C) Fmoc-Lys-GA(5)A-GC(1O2)C-A(5)GG-C(1O2)AA(5)-G-Lys-NH₂
(N ----+ C) Fmoc-GA(5)A-GC(1O2)C-A(5)GG-C(1O2)AA(5)-G-Lys-NH₂;
(N → C) Fmoc-Gly-GA(5)A-GC(1O2)C-A(5)GG-C(1O2)AA(5)-G-NH₂;
(N → C) Fmoc-Lys-Gly-GA(5)A-GC(1O2)C-A(5)GG-C(1O2)AA(5)-G-Lys-NH₂;
(N → C) Fmoc-Val-Gly-GA(5)A-GC(1O2)C-A(5)GG-C(1O2)AA(5)-G-NH₂;
(N → C) Fmoc-GA(6)A-GC(1O2)C-A(6)GG-C(1O2)AA(6)-G-NH₂;
(N → C) Fmoc-G(6)AA(5)-GC(1O3)C-A(7)GG(5)-CA(5)A-G-NH₂;
(N → C) Fmoc-GA(5)A-GC(2O2)C-A(6)GG-C(1O5)AA(6)-G-NH₂;
(N → C) Fmoc-TG(6)C(1O5)-GGA(6)-AG(6)C-CA(6)G-GC(1O2)A-A(6)GG(6)-HN₂;
(N → C) Fethoc-C(1O2)TT-A(5)CC-A(5)GG-C(1O2)AA(5)-G-NH₂;
(N → C) Fethoc-C(1O2)TT-A(5)CC-A(5)GG-C(1O2)AA(5)-G-Lys-Lys-NH₂;
(N → C) Fethoc-C(1O2)TT-A(6)CC-A(5)GG-C(1O3)AA(5)-G-Val-Lys-NH₂;
(N → C) Ac-C(1O2)TT-A(5)CC-A(5)GG-C(1O2)TA(5)-G-NH₂;
(N → C) Piv-C(1O2)TT-A(5)CC-A(5)GG-C(1O2)AA(5)-G-NH₂;
(N → C) H-C(1O2)TT-A(5)CC-A(5)GG-C(1O2)AA(5)-G-NH₂;
(N → C) H-CTT-A(5)C(1O3)C-A(5)G(3)G-C(1O2)AA(5)-G-NH₂;
(N → C) n-Propyl-C(1O2)TT-A(5)CC-A(5)GG-C(1O2)AA(5)-G-NH₂;
(N → C) n-Propyl-CTT-A(5)C(2O2)C-A(3)G(2O3)G-C(1O2)AA(5)-G-NH₂;
(N → C) p-Toluenesulfonyl-CTT-A(5)C(1O2)C-A(8)G(5)G-C(1O2)AA(5)-G-NH₂;
(N → C) Benzoyl-Lys-Val-C(1O2)TT-A(5)CC-A(5)GG-C(1O2)AA(5)-G-NH₂;
(N → C) Benzoyl-CTT-A(5)C(1O5)C-A(5)G(2O2)G-C(1O2)AA(5)-G-NH₂;
(N → C) Fethoc-Lys-Leu-CTT-A(5)C(1O2)C-A(2O2)GG-C(1O2)AA(5)-G-Lys-NH₂;
(N → C) Fethoc-C(1O2)TT-A(5)CC-A(5)GG-C(1O5)AA(5)-G-NH₂;
(N → C) Fethoc-C(1O2)TT-A(5)CC-A(5)GT-C(1O2)AA(5)-G-NH₂;
(N → C) Fethoc-C(1O2)TT-A(5)CC-A(5)GG-C(1O2)TA(5)-G-NH₂;
(N → C) Fethoc-C(1O2)TT-A(5)CC-A(5)GG-C(1O2)A(5)A-G-NH₂;
(N → C) Fethoc-C(1O2)TT-A(5)CC-AG(5)G-C(1O2)AA(5)-G-NH₂;
(N → C) Fethoc-C(1O2)TT-A(5)CC-A(7)GG-C(1O2)AA(3)-G-NH₂;
(N → C) Fethoc-CTT-A(5)C(1O2)C-A(5)GT-C(1O2)TA(5)-G-NH₂;
(N → C) Fethoc-CTT-A(5)C(1O2)C-A(5)GT-C(1O2)TA(5)-G-Arg-NH₂;
(N → C) Fethoc-TC(1O2)C-TTA(6)-CCA(6)-GGC(1O2)-AA(6)G-G(6)-NH₂;
(N → C) Fethoc-TC(1O2)C-TTA(5)-CCA(5)-GGC(1O2)-AA(5)G-G(6)-NH₂;
(N → C) Fethoc-GA(5)T-AC(1O2)C-A(5)GG(6)-CAA(5)-G-NH₂;
(N → C) Fethoc-TA(5)C-CAG(6)-GC(1O2)A-A(5)GG(6)-C-NH₂;
(N → C) Fethoc-C(1O2)TT-A(5)CC-A(5)GG(6)-CA(5)A-NH₂;
(N → C) Benzyl-C(1O2)TT-A(2O2)CC-A(5)GG(6)-CA(5)A-NH₂;
(N → C) Phenyl-C(1O2)TT-A(5)CC-A(5)GG(6)-CA(5)A-NH₂;
(N → C) Fethoc-C(1O2)TT-A(5)CC-A(7)GG(5)-CA(5)A-NH₂;
(N → C) Fethoc-C(1O2)TT-A(5)CC-A(6)GG(2O2)-CA(5)A-NH₂;
(N → C) Fethoc-C(1O2)TT-A(5)CC-A(5)GG(6)-CA(5)A-Val-Lys-NH₂;
(N → C) Piv-C(1O2)TT-A(5)CC-A(5)GG(6)-CA(5)A-NH₂;
(N → C) Fmoc-Lys-Val-C(1O2)TT-A(5)CC-A(5)GG(6)-CA(5)A-NH₂;
(N → C) Ac-C(1O2)TT-A(5)CC-A(5)GG(6)-CA(5)A-NH₂;
(N → C) H-C(1O2)TT-A(5)CC-A(5)GG(6)-CA(5)A-Lys-NH₂;
(N → C) Piv-Arg-C(1O2)TT-A(5)CC-A(5)GG(6)-CA(5)A-Lys-NH₂;
(N → C) N-Phenyl-N-methyl-CTT-A(5)C(1O2)C-A(5)GG-C(1O2)AA(5)-G-Lys-NH₂;
(N → C) [N-(2-Phenylethyl)amino]carbonyl-CTT-A(5)C(1O2)C-A(4)G(5)G-C(1O2)AA(5)-G-NH₂;
(N → C) Benzoyl-Leu-C(1O2)TT-A(5)CC-A(5)GG(6)-CA(5)A-Lys-NH₂;
(N → C) Fethoc-C(1O3)TT-A(5)CC-A(5)GG(5)-CA(5)A-NH₂;
(N → C) Fethoc-C(1O2)TT-A(6)CC-A(6)GG(6)-CA(6)A-NH₂;
(N → C) Fethoc-TTT-TCC(1O2)-TTA(6)-CCA(6)-GG(6)C-A(6)A-NH₂;
(N → C) Fethoc-TTT-TCC(1O2)-TTA(6)-CC(1O3)A(6)-G-Lys-NH₂;
(N → C) Fethoc-TC(2O2)C-TTA(6)-CCA(6)-GG(6)C-A(6)A-NH₂;
(N → C) Me-Gly-TC(2O2)C-TTA(6)-CCA(6)-GG(6)C-A(6)A-NH₂;
(N → C) Fethoc-Lys-TTT-TCC(1O2)-TTA(6)-CCA(6)-GG(6)C-NH₂; and
(N → C) Fethoc-Arg-TCC(1O2)-TTA(5)-CCA(6)-GG(5)C-Lys-NH₂:
   wherein,
   A, G, T, and C are PNA monomers with a natural nucleobase of adenine, guanine, thymine, and cytosine, respectively;
   C(pOq), A(p), A(pOq), G(p), and G(pOq) are PNA monomers with an unnatural nucleobase represented by **Formula VI, Formula VII, Formula VIII, Formula IX,** and **Formula X,** respectively; 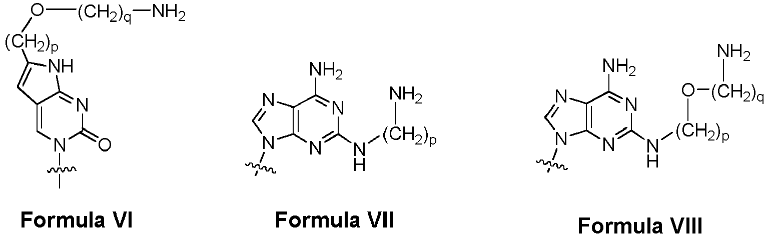 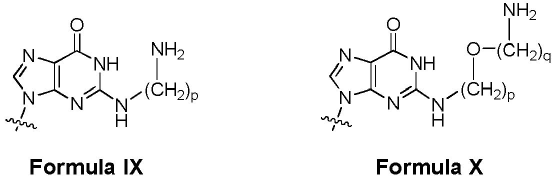 wherein,
      p and q are integers; and,
      the abbreviations for the N- and C-terminus substituents are as specifically described as follows: "Fmoc-" is the abbreviation for "[(9-fluorenyl)methyloxy]carbonyl-"; "Fethoc-" for "[2-(9-fluorenyl)ethyl-1-oxy]carbonyl"; "Ac-" for "acetyl-"; "Benzoyl-" for "benzenecabonyl-"; "Piv-" for "pivalyl-"; "Methyl-" for "methyl-"; "n-Propyl-" for "1-(n-propyl)-"; "H-" for "hydrido-" group; "p-Toluenesulfonyl" for "(4-methylbenzene)-1-sulfonyl-"; "-Lys-" for amino acid residue "lysine"; "-Val-" for amino acid residue "valine"; "-Leu-" for amino acid residue "leucine"; "-Arg-" for amino acid residue "arginine"; "-Gly-" for amino acid residue "glycine"; "[N-(2-Phenylethyl)amino]carbonyl-" for "[N-1-(2-phenylethyl)amino]carbonyl-"; "Benzyl-" for "1-(phenyl)methyl-"; "Phenyl-" for "phenyl- "; "Me-" for "methyl-"; and "-NH₂" for non-subsituted "-amino" group.

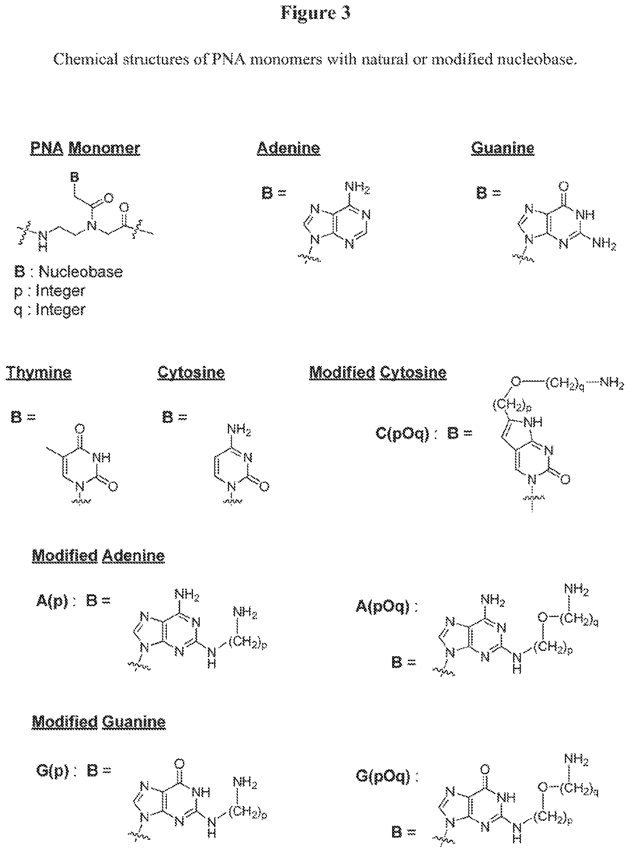

Figure 3 collectively and unambiguously provides the chemical structures for the PNA monomers abbreviated as A, G, T, C, C(pOq), A(p), A(pOq), G(p), and G(pOq). As discussed in the prior art [PCT/KR2009/001256, published as WO 2009/113828], C(pOq) is regarded as a "modified cytosine" PNA monomer due to its hybridization for "guanine". A(p) and A(pOq) are taken as "modified adenine" PNA monomers for their hybridization for "thymine". Likewise G(p) and G(pOq) are considered to be "modified guanine" PNA monomers for their base pairing with "cytosine".

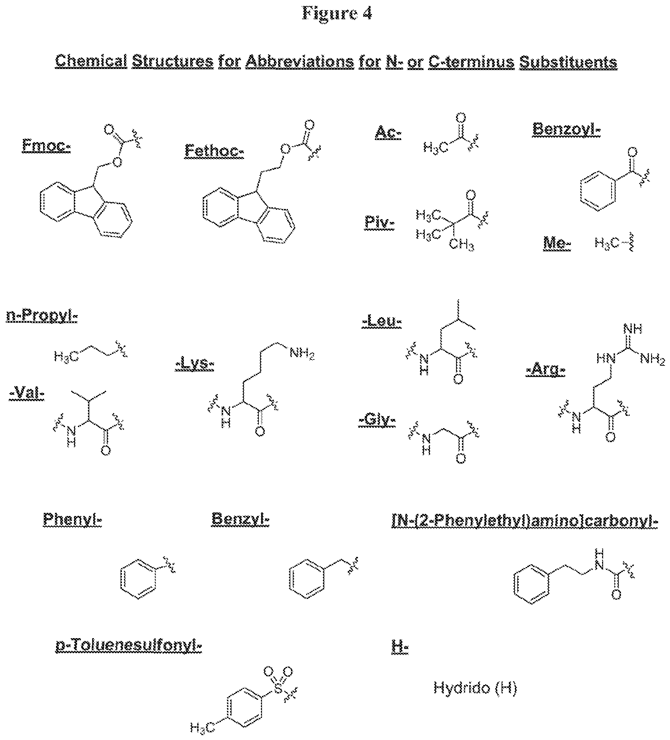

Figure 4 unequivocally illustrates the chemical structures for a variety of abbreviations for substituents used for diversifying the N-terminus or C-terminus of the PNA derivative of **Formula I** in this invention.

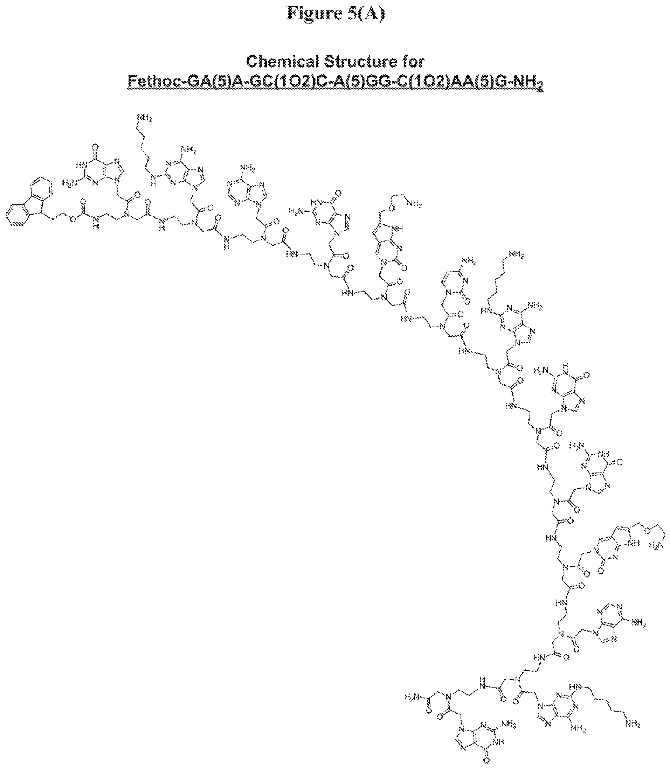

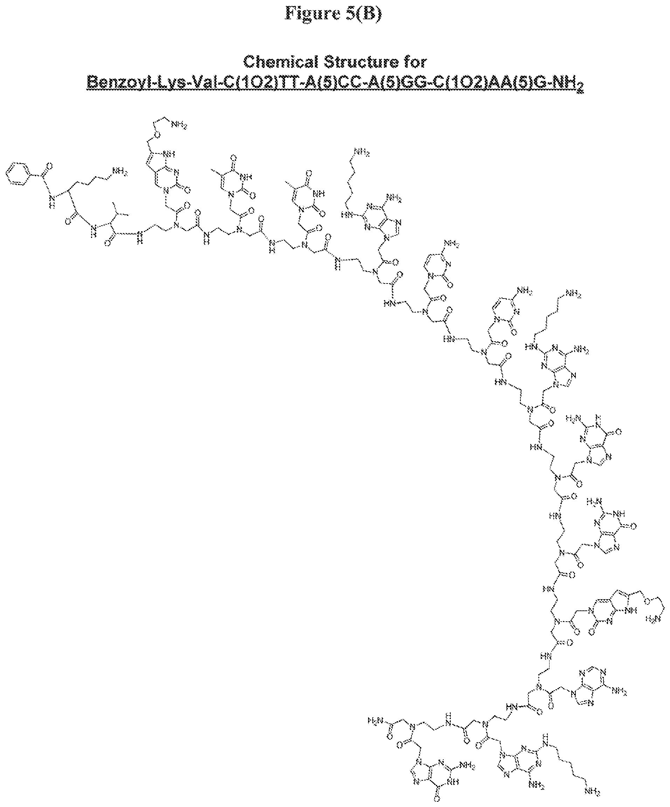

In order to illustrate the abbreviations for the PNA derivatives, the chemical structure for the PNA derivative abbreviated as "(N —> C) Fethoc-GA(5)A-GC(1O2)C-A(5)GG-C(1O2)AA(5)-G-NH₂" is provided in Figure 5(A). As another illustration, the chemical structure for the PNA derivative abbreviated as "(N ----+ C) Benzoyl-Lys-Val-C(1O2)TT-A(5)CC-A(5)GG-C(1O2)AA(5)-G-NH₂" is provided in Figure 5(B).

The 13-mer PNA sequence of "(N —> C) Fethoc-GA(5)A-GC(1O2)C-A(5)GG-C(1O2)AA(5)-G-NH₂" is equivalent to the DNA sequence of "(5' —> 3') GAA-GCC-AGG-CAA-G" in binding to its complementary binding with pre-mRNA. The 13-mer PNA has a 9-mer complementary overlap with the 9-mer sequence marked as "bold" and "underlined" in the 20-mer RNA sequence [(5' —> 3') GC**CUUGCCUG** | **g**"uaag"gaaaa] spanning the junction of "exon 5" and "intron 5" within the human AR pre-mRNA. It is noted that the four single mismatches in "intron 5" is marked as "uaag".

The 13-mer PNA sequence of "(N —> C) Benzoyl-Lys-Val-C(1O2)TT-A(5)CC-A(5)GG-C(1O2)AA(5)-G-NH₂" is equivalent to the DNA sequence of "(5' —> 3') CTT-ACC-AGG-CAA-G", which has a 13-mer complementary overlap with the 13-mer sequence marked as "bold" and "underlined" in the 20-mer RNA sequence [(5' —> 3') GC**CUUGCCUG** | **guaag**gaaaa] spanning the junction of "exon 5" and "intron 5" within the human AR pre-mRNA.

The 13-mer PNA sequence of "(N ----+ C) Ac-C(1O2)TT-A(5)CC-A(5)GG-C(1O2)TA(5)-G-NH₂" is equivalent to the DNA sequence of "(5' —> 3') CTT-ACC-AGG-CTA-G", which has a 12-mer complementary overlap with the 12-mer sequence as marked "bold" and "underlined" in the 20-mer RNA sequence [(5' → 3') GC**CU**"U"**GCCUG** | **guaag**gaaaa] spanning the junction of "exon 5" and "intron 5" within the human AR pre-mRNA. It is noted that the single mismatch in exon 5 is marked as "U".

The 17-mer PNA sequence of "(N ----+ C) Fethoc-TTT-TCC(1O2)-TTA(6)-CCA(6)-GG(6)C-A(6)A-NH₂" is equivalent to the DNA sequence of "(5' ----+ 3') TTT-TCC-TTA-CCA-GGC-AA", which has a 17-mer complementary overlap with the 17-mer sequence marked as "bold" and "underlined" in the 20-mer RNA sequence [(5' —> 3') GCC**UUGCCUG** | **guaaggaaaa** ] spanning the junction of "exon 5" and "intron 5" within the human AR pre-mRNA.

The present invention provides a PNA derivative of **Formula I** which is selected from the group of specifically preferred compounds enlisted below, or a pharmaceutically acceptable salt thereof:
(N → C) Fmoc-GA(5)A-GC(1O2)C-A(5)GG-C(1O2)AA(5)-G-NH₂;
(N → C) Fethoc-GA(5)A-GC(1O2)C-A(5)GG-C(1O2)AA(5)-G-NH₂;
(N → C) Fmoc-GA(6)A-GC(102)C-A(6)GG-C(102)AA(6)-G-NH₂;
(N → C) Fmoc-G(6)AA(5)-GC(103)C-A(7)GG(5)-CA(5)A-G-NH₂;
(N → C) Fmoc-GA(5)A-GC(2O2)C-A(6)GG-C(1O5)AA(6)-G-NH₂;
(N → C) Fethoc-TG(6)C(1O2)-GGA(6)-AG(6)C-CA(6)G-GC(1O2)A-A(6)GG(6)-NH₂;
(N → C) Fethoc-C(102)TT-A(5)CC-A(5)GG-C(102)AA(5)-G-NH₂;
(N → C) Fethoc-C(102)TT-A(6)CC-A(5)GG-C(103)AA(5)-G-Val-Lys-NH₂;
(N → C) Ac-C(102)TT-A(5)CC-A(5)GG-C(102)TA(5)-G-NH₂;
(N → C) Piv-C(102)TT-A(5)CC-A(5)GG-C(102)AA(5)-G-NH₂;
(N → C) H-CTT-A(5)C(103)C-A(5)G(3)G-C(102)AA(5)-G-NH₂;
(N → C) n-Propyl-C(102)TT-A(5)CC-A(5)GG-C(102)AA(5)-G-NH₂;
(N → C) n-Propyl-CTT-A(5)C(202)C-A(3)G(203)G-C(102)AA(5)-G-NH₂;
(N → C) p-Toluenesulfonyl-CTT-A(5)C(102)C-A(8)G(5)G-C(102)AA(5)-G-NH₂;
(N → C) Benzoyl-CTT-A(5)C(105)C-A(5)G(202)G-C(102)AA(5)-G-NH₂;
(N → C) Fethoc-Lys-Leu-CTT-A(5)C(1O2)C-A(2O2)GG-C(1O2)AA(5)-G-Lys-NH₂;
(N → C) Fethoc-C(102)TT-A(5)CC-A(5)GG-C(105)AA(5)-G-NH₂;
(N → C) Fethoc-C(102)TT-A(5)CC-A(7)GG-C(102)AA(3)-G-NH₂;
(N → C) Fethoc-CTT-A(5)C(102)C-A(5)GT-C(102)TA(5)-G-NH₂;
(N → C) Fethoc-TC(102)C-TTA(6)-CCA(6)-GGC(102)-AA(6)G-G(6)-NH₂;
(N → C) Fethoc-TC(102)C-TTA(5)-CCA(5)-GGC(102)-AA(5)G-G(6)-NH₂;
(N → C) Fethoc-GA(5)T-AC(102)C-A(5)GG(6)-CAA(5)-G-NH₂;
(N → C) Fethoc-TA(5)C-CAG(6)-GC(1O2)A-A(5)GG(6)-C-NH₂;
(N → C) Fethoc-C(102)TT-A(5)CC-A(5)GG(6)-CA(5)A-NH₂;
(N → C) Fethoc-C(102)TT-A(5)CC-A(7)GG(5)-CA(5)A-NH₂;
(N → C) Fethoc-C(102)TT-A(5)CC-A(6)GG(202)-CA(5)A-NH₂;
(N → C) Piv-C(102)TT-A(5)CC-A(5)GG(6)-CA(5)A-NH₂;
(N → C) Ac-C(102)TT-A(5)CC-A(5)GG(6)-CA(5)A-NH₂;
(N → C) N-Phenyl-N-methyl-CTT-A(5)C(1O2)C-A(5)GG-C(1O2)AA(5)-G-Lys-NH₂;
(N → C) [N-(2-Phenylethyl)amino]carbonyl-CTT-A(5)C(1O2)C-A(4)G(5)G-C(102)AA(5)-G-NH₂;
(N → C) Fethoc-C(102)TT-A(6)CC-A(6)GG(6)-CA(6)A-NH₂;
(N → C) Fethoc-TTT-TCC(102)-TTA(6)-CCA(6)-GG(6)C-A(6)A-NH₂;
(N → C) Fethoc-TTT-TCC(1O2)-TTA(6)-CC(1O3)A(6)-G-Lys-NH₂;and
(N → C) Fethoc-TC(2O2)C-TTA(6)-CCA(6)-GG(6)C-A(6)A-NH₂.

Also provided are a peptide nucleic acid derivative selected from the group consisting of: (N → C) Fethoc-C(1O2)TT-A(5)CC-A(5)GG-C(1O2)AA(5)-G-NH₂, (N → C) Fethoc-TC(1O2)C-TTA(6)-CCA(6)-GGC(1O2)-AA(6)G-G(6)-NH₂, (N → C) Fethoc-TC(1O2)C-TTA(5)-CCA(5)-GGC(1O2)-AA(5)G-G(6)-NH₂, (N → C) Fethoc-TA(5)C-CAG(6)-GC(1O2)A-A(5)GG(6)-C-NH₂, (N → C) Fethoc-C(1O2)TT-A(5)CC-A(5)GG(6)-CA(5)A-NH₂, (N → C) Fethoc-C(1O2)TT-A(5)CC-A(6)GG(6)-CA(5)A-NH₂, (N → C) Ac-C(1O2)TT-A(5)CC-A(5)GG(6)-CA(5)A-NH₂, or (N → C) Fethoc-C(1O2)TT-A(6)CC-A(6)GG(6)-CA(6)A-NH₂, or a pharmaceutically acceptable salt thereof.

### Detailed Description of Invention

### General Procedures for Preparation of PNA Oligomers

PNA oligomers were synthesized by solid phase peptide synthesis (SPPS) based on Fmoc-chemistry according to the method disclosed in the prior art [US6,133,444; WO96/40685] with minor but due modifications. The solid support employed in this study was H-Rink Amide-ChemMatrix purchased from PCAS BioMatrix Inc. (Quebec, Canada). Fmoc-PNA monomers with a modified nucleobase were synthesized as described in the prior art [PCT/KR 2009/001256, published as WO 2009/113828] or with minor modifications. Such Fmoc-PNA monomers with a modified nucleobase and Fmoc-PNA monomers with a naturally occurring nucleobase were used to synthesize the PNA derivatives of the present invention. PNA oligomers were purified by C₁₈-reverse phase HPLC (watre/acetonitrile or water/methanol with 0.1% TFA) and characterized by mass spectrometry.

Scheme 1 illustrates a typical monomer elongation cycle adopted in the SPPS of this invention, and procedural details are provided below. To a skilled person in the field, however, lots of minor variations are obviously possible in effectively running such SPPS reactions on an automatic peptide synthesizer or manual peptide synthesizer. Each reaction step in Scheme 1 is briefly provided as follows.

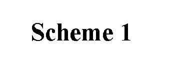

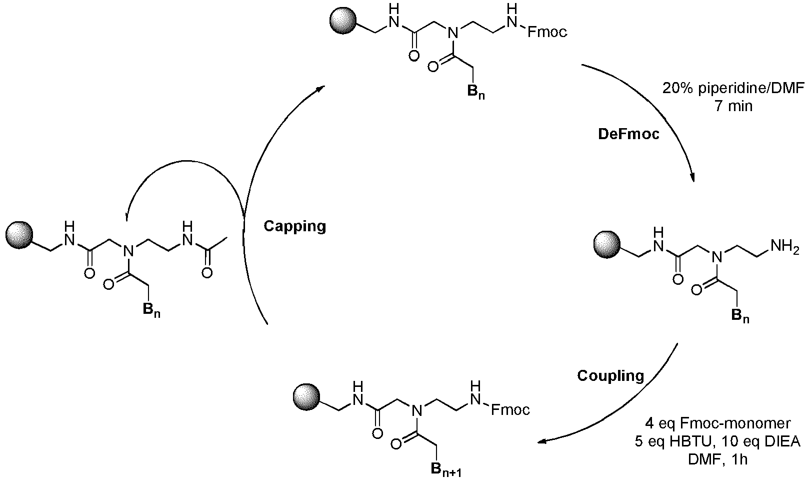

[Activation of H-Rink-ChemMatrix Resin] 0.01 mmol (ca 20 mg resin) of the ChemMatrix resin in 1.5 mL 20% piperidine/DMF was vortexed in a libra tube for 20 min, and the DeFmoc solution was filtered off. The resin was washed for 30 sec each in series with 1.5 mL methylene chloride (MC), 1.5 mL dimethylformamide (DMF), 1.5 mL MC, 1.5 mL DMF, and 1.5 mL MC. The resulting free amines on the solid support were subjected to coupling either with an Fmoc-PNA monomer or with an Fmoc-protected amino acid derivative.

[DeFmoc] The resin was vortexed in 1.5 mL 20% piperidine/DMF for 7 min, and the DeFmoc solution was filtered off. The resin was washed for 30 sec each in series with 1.5 mL MC, 1.5 mL DMF, 1.5 mL MC, 1.5 mL DMF, and 1.5 mL MC. The resulting free amines on the solid support were immediately subjected to coupling with an Fmoc-PNA monomer.

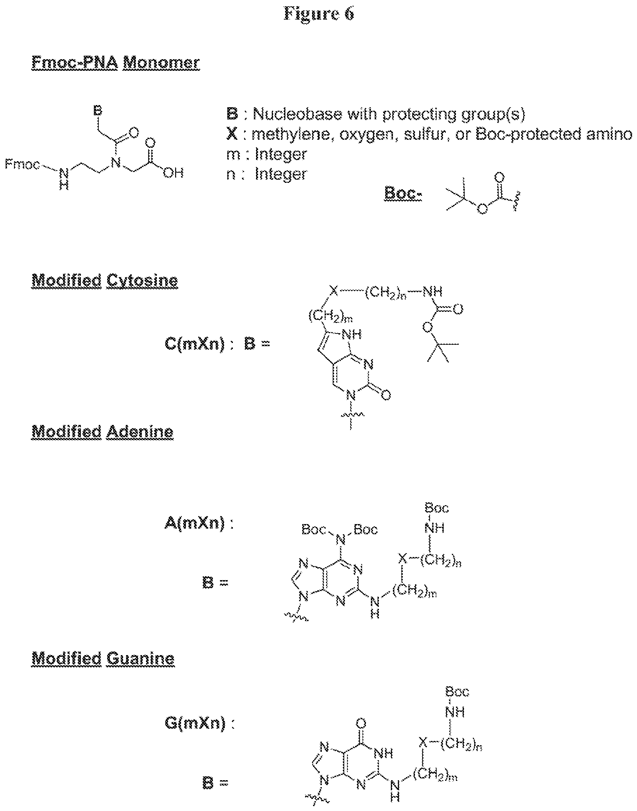

[Coupling with Fmoc-PNA Monomer] The free amines on the solid support were coupled with an Fmoc-PNA monomer as follows. 0.04 mmol of PNA monomer, 0.05 mmol HBTU, and 10 mmol DIEA were incubated for 2 min in 1 mL anhydrous DMF, and added to the resin with free amines. The resin solution was vortexed for 1 hour and the reaction medium was filtered off. Then the resin was washed for 30 sec each in series with 1.5 mL MC, 1.5 mL DMF, and 1.5 mL MC. The chemical structures of Fmoc-PNA monomers with a modified nucleobase used in this invention are provided in Figure 6. The Fmoc-PNA monomers with a modified nucleobase are provided in Figure 6 should be taken as examples, and therefore should not be taken to limit the scope of the present invention. A skilled person in the field may easily figure out a number of variations in Fmoc-PNA monomers to synthesize the PNA derivative of **Formula I.**

[Capping] Following the coupling reaction, the unreacted free amines were capped by shaking for 5 min in 1.5 mL capping solution (5% acetic anhydride and 6% 2,6-leutidine in DMF). Then the capping solution was filtered off and washed for 30 sec each in series with 1.5 mL MC, 1.5 mL DMF, and 1.5 mLMC.

[Introduction of "Fethoc-" Radical in N-Terminus] "Fethoc-" radical was introduced to the N-terminus by reacting the free amine on the resin with "Fethoc-OSu" under basic coupling conditions. The chemical structure of "Fethoc-OSu" [CAS No. 179337-69-0, C₂₀H₁₇NO₅, MW 351.36] is provided as follows.

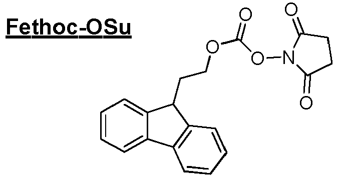

[Cleavage from Resin] PNA oligomers bound to the resin were cleaved from the resin by shaking for 3 hours in 1.5 mL cleavage solution (2.5% tri-isopropylsilane and 2.5% water in trifluoroacetic acid). The resin was filtered off and the filtrate was concentrated under reduced pressure. The residue was triturated with diethylether, and the resulting precipitate was collected by filtration for purification by reverse phase HPLC.

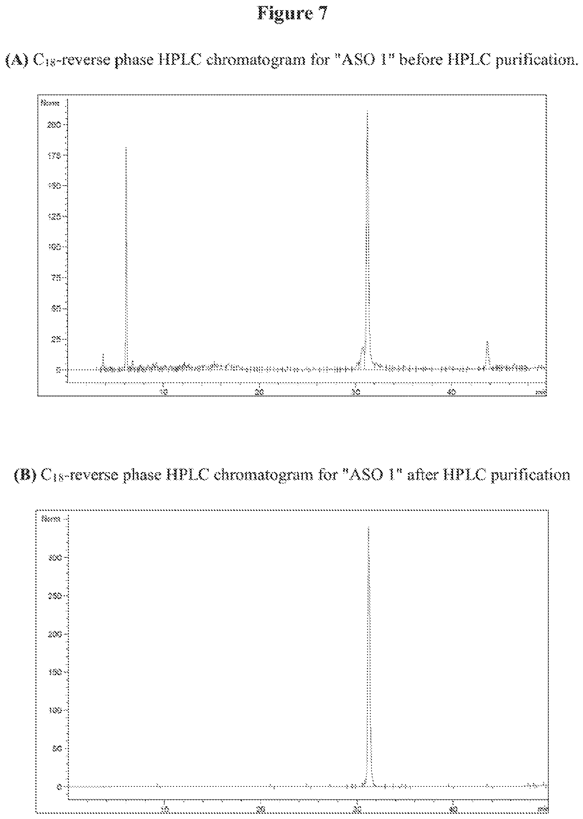

[HPLC Analysis and Purification] Following a cleavage from resin, the crude product of a PNA derivative was purified by C₁₈-reverse phase HPLC eluting water/acetonitrile or water/methanol (gradient method) containing 0.1% TFA. Figures 7(A) and 7(B) are exemplary HPLC chromatograms for "ASO 1" before and after HPLC purification, respectively. The oligomer sequence of "ASO 1" is as provided in Table 1.

### Synthetic Examples for PNA Derivatives of Formula I

PNA derivatives of this invention were prepared according to the synthetic procedures provided above or with minor modifications. Table 1 provides examples of AR ASOs of the present invention along with structural characterization data by mass spectrometry. Provision of the AR ASOs in Table 1 is to exemplify the PNA derivatives of **Formula I,** and should not be interpreted to limit the scope of the present invention.

**Table 1. PNA derivatives of Formula I and structural identification by mass spectrometry.**

| PNA Example | PNA Sequence (N → C) | Exact Mass, m/z | |
|---|---|---|---|
| | | theor.^{a} | obs.^{b} |
| ASO 1 | Fmoc-GA(5)A-GC(1O2)C-A(5)GG-C(1O2)AA(5)-G- NH2 | 4317.91 | 4317.96 |
| ASO 2 | Fethoc-GA(5)A-GC(1O2)C-A(5)GG-C(1O2)AA(5)-G- NH2 | 4331.93 | 433'.97 |
| ASO 3 | Fmoc-TG(6)C(1O2)-GGA(6)-AG(6)C-CA(6)G- GC(1O2)A-A(6)GG(6)-NH2 | 6047.80 | 6047.76 |
| ASO 4 | Fmoc-GA(6)A-GC(1O2)C-A(6)GG-C(1O2)AA(6)-G- NH2 | 4359.96 | 4356.96 |
| ASO 5 | Fethoc-C(1O2)TT-A(5)CC-A(5)GG-C(1O2)AA(5)-G- NH2 | 4257.90 | 4257.92 |
| ASO 6 | Fethoc-TC(1O2)C-TTA(6)-CCA(6)-GGC(1O2)- AA(6)G-G(6)-NH2 | 5207.37 | 5207.42 |
| ASO 7 | Fethoc-TC(1O2)C-TTA(5)-CCA(5)-GGC(1O2)- AA(5)G-G(6)-NH2 | 5165.32 | 5165.31 |
| ASO 8 | Fethoc-GA(5)T-AC(1O2)C-A(5)GG(6)-CAA(5)-G-NH₂ | 4308.97 | 4308.98 |
| ASO 9 | Fethoc-TA(5)C-CAG(6)-GC(1O2)A-A(5)GG(6)-C-NH₂ | 4283.97 | 4283.96 |
| ASO 10 | Fethoc-C(1O2)TT-A(5)CC-A(5)GG(6)-CA(5)A-NH₂ | 3968.85 | 3968.86 |
| ASO 11 | Fethoc-C(1O2)TT-A(5)CC-A(6)GG(6)-CA(5)A-NH₂ | 3982.86 | 3982.88 |
| ASO 12 | Ac-C(1O2)TT-A(5)CC-A(5)GG(6)-CA(5)A-NH₂ | 3774.77 | 3774.83 |
| ASO 13 | Fethoc-C(1O2)TT-A(6)CC-A(6)GG(6)-CA(6)A-NH₂ | 4010.89 | 4010.93 |
| ASO 14 | H-CTT-A(5)C(1O3)C-A(5)G(3)G-C(1O2)AA(5)-G-NH₂ | 4092.89 | 4092.90 |
| ASO 15 | Benzoyl-CTT-A(5)C(1O5)C-A(5)G(2O2)G- C(1O2)AA(5)-G-NH2 | 4254.96 | 4254.99 |
| ASO 16 | n-Propyl-CTT-A(5)C(2O2)C-A(3)G(2O3)G- C(1O2)AA(5)-G-NH2 | 4150.93 | 4150.93 |
| ASO 17 | p-Toluenesulfonyl-CTT-A(5)C(1O2)C-A(8)G(5)G- C(1O2)AA(5)-G-NH2 | 4302.96 | 4302.90 |
| ASO 18 | Fethoc-Lys-Leu-CTT-A(5)C(1O2)C-A(2O2)GG- C(1O2)AA(5)-G-Lys-NH2 | 4629.16 | 4629.16 |
| ASO 19 | Fethoc-CTT-A(5)C(1O2)C-A(5)GT-C(1O2)TA(5)-G- NH2 | 4223.88 | 4223.93 |
| ASO 20 | N-Phenyl-N-methyl-CTT-A(5)C(1O2)C-A(5)GG- C(1O2)AA(5)-G-Lys-NH2 | 4239.96 | 4240.00 |
| ASO 21 | [N-(2-Phenylethyl)amino]carbonyl-CTT-A(5)C(1O2)C- A(4)G(5)G-C(1O2)AA(5)-G-NH2 | 4239.96 | 4239.95 |

| | | | |
|---|---|---|---|
| ^{a)} theoretical exact mass, ^{b)} observed exact mass | | | |

### Binding Affinity of PNA for 10-mer Complementary DNA

The PNA derivatives in Table 1 were evaluated for their binding affinity for 10-mer DNAs complementarily targeting either the N-terminal or C-terminal. The binding affinity was assessed by Tₘ value for the duplex between PNA and 10-mer complementary DNA. The duplex between PNA derivatives in Table 1 and fully complementary DNAs show Tₘ values too high to be reliably determined in aqueous buffer solution, since the buffer solution tends to boil off during the Tₘ measurement.

Tₘ values were determined on an UV/Vis spectrometer as follows. A mixed solution of 4 µM PNA oligomer and 4 µM complementary 10-mer DNA in 4 mL aqueous buffer (pH 7.16, 10 mM sodium phosphate, 100 mM NaCl) in 15 mL polypropylene falcon tube was incubated at 90°C for a minute and slowly cooled down to ambient temperature over several minutes. Then the solution was transferred into a 3 mL quartz UV cuvette equipped with an air-tight cap, and subjected to a Tₘ measurement at 260 nm on an Agilent 8453 UV/Visible spectrophotometer or a similar one as described in the prior art [PCT/KR2009/001256, published as WO 2009/113828] or with minor modifications. The 10-mer complementary DNAs for Tₘ measurement were purchased from Bioneer (www.bioneer.com, Dajeon, Republic of Korea) and used without further purification.

Observed Tₘ values of the PNA derivatives of **Formula I** are very high for a complementary binding to 10-mer DNA, and provided in Table 2. For example, "ASO 5" showed a Tₘ value of 86.1°C for the duplex with the 10-mer complementary DNA targeting the N-terminal 10-mer within the PNA marked as "bold" and "underlined" in [(N → C) Fethoc-**C(1O2)TT-A(5)CC-A(5)GG-C(1O2)**AA(5)-G-NH₂]. In the meantime, "ASO 5" showed a Tₘ of 81.3°C for the duplex with the 10-mer complementary DNA targeting the C-terminal 10-mer within the PNA marked as "bold" and "underlined" in [(N → C) Fethoc-C(102)TT-**A(5)CC-A(5)GG-C(102)AA(5)-G**-NH₂].

**Table 2. Tₘ values between PNAs in Table and 10-mer complementary DNA targeting either the N-terminal or the C-terminal of PNA.**

| PNA | Tₘ Value, °C | |
|---|---|---|
| | 10-mer DNA against N-Terminal | 10-mer DNA against C-Terminal |
| ASO 5 | 86.1 | 81.3 |
| ASO 8 | 87.4 | 86.0 |
| ASO 9 | 84.3 | 84.5 |
| ASO 10 | 84.4 | 78.4 |
| ASO 13 | 83.0 | 87.0 |

### Examples for Biological Activities for PNA Derivatives of Formula I

PNA derivatives of **Formula I** were evaluated for their biological activities in vitro and in vivo. The biological examples provided below are provided as examples to illustrate the biological profiles of such PNA derivatives of **Formula I,** and therefore should not be interpreted to limit the scope of the current invention.

### Example 1. Exon Skipping Induced by "ASO 5".

"ASO 5" specified in Table 1 is a 13-mer antisense oligonucleotide which has a 13-mer full complementary overlap with the 13-mer sequence marked as "bold" and "underlined" in the 20-mer RNA sequence [(5' —> 3') GC**CUUGCCUG** | **guaag**gaaaa] spanning the junction of "exon 5" and "intron 5" within the human AR pre-mRNA.

"ASO 5" was evaluated by AR nested PCR for its ability to induce the skipping of AR "exon 5" in MCF7 cells (Cat. Number: HTB-22, ATCC). The employed procedures are detailed as follows.

[Cell Culture & ASO Treatment] MCF7 cells were grown in EMEM medium supplemented with 10% FBS, 1% streptomycin/penicillin, and 0.01 mg/ml bovine insulin under 5% CO₂ atmosphere at 37°C. Cells were sub-cultured in 60 mm culture dish prior to treatment with "ASO 5" at 3 aM to 3 fM.

[RNA Extraction] MCF7 cells were incubated with or without "ASO 5" for 3 hours. Total RNA was extracted from cells in 60 mm culture dish using "Universal RNA Extraction Kit" (Cat. No. 9767, Takara) according to the manufacturer's instructions

[cDNA Synthesis by One-step PCR] 100 ng of RNA template was used in a 25 µL reverse transcription reaction using Super Script^{®} One-Step RT-PCR kit with platinum^{®} Taq polymerase (Cat. No. 10928-042, Invitrogen) against a set of gene-specific primers [exon 3_forward: (5' → 3') TGGGTGTCACTATGGAGC, and exon 9_reverse: (5' → 3') GGGTGT-GGAAATAGATGGG] according to the following cycle conditions: 50°C for 30 min and 94°C for 2 min, followed by 39 cycles of 30 sec at 94°C, 30 sec at 55 °C, and 1 min at 72°C.

[Nested PCR Amplification] Throughout the amplification process, was used a unique amplification technique (touch up as increasing annealing temperature per cycle) that worked efficiently and specifically over a temperature range, rather than at one specific annealing temperature (i.e. conventional PCR method). 1 µL of cDNA was further amplified in a 20 µL nested PCR (Invitrogen) reaction against a set of primers [exon 3_forward: (5' →

3') TGGGTG-TCACTATGGAGC, and exon 7n_reverse: (5' → 3') GGGGTGATTTGGAGCCAT] according to the following cycle conditions: initial 10 cycles [94°C for 30 sec, 47°C for 40 sec (+0.5°C every cycle), 72°C for 40 sec], followed by 20 cycles [94°C for 30 sec, 50°C for 30 sec, and 72°C for 40 sec].

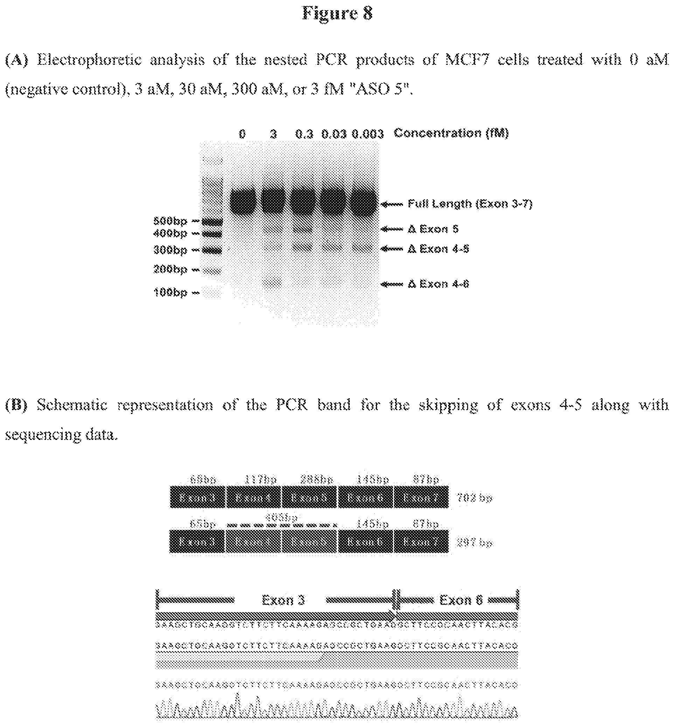

[Identification of Exon Skipping Products] The PCR products were subjected to electrophoretic separation on a 2% agarose gel. The bands of target size were collected and analyzed by Sanger Sequencing. In Figure 8(A), there were three treatment-related PCR product bands assignable to AR mRNA splice variants lacking "exon 5". "ASO 5" was found to induce the skipping of "exon 5", "exons 4-5", and "exons 4-6", although the ratio of the skipping products appeared to be dependent on the ASO concentration. Figure 8(B) provides the actual sequencing data for the skipping band of "exons 4-5" in Figure 8(A) as an example for Sanger Sequencing.

### Example 2. qPCR Evaluation of AR mRNA Level in MCF7 Cells treated with "ASO 5".

"ASO 5" was evaluated for its ability to down-regulate the human AR mRNA by qPCR with SYBR Green detection.

MCF7 cells were sub-cultured in 5 mL culture medium in 60 mm culture dish, and treated or with "ASO 5" at 0 zM (negative control) to 1 aM (2 culture dishes per each concentration). 5 hours later, total RNA was extracted with "MiniBEST Universal RNA Extraction Kit" according to the manufacturer's instructions (Cat. No. 9767, Takara). 500 ng of RNA template were used to synthesize cDNA for a 50 µL reverse transcription reaction using Oligo-dT according to the manufacturer's instructions (Cat. No. 6110A, Takara). cDNA was then subjected to the 1^{st} PCR against a set of primers covering "exon 3" to "exon 9" [Exon 3_forward: (5' → 3') TGGGTGTCACTATGGAGC, and Exon 9_reverse: (5' → 3') GGGTG-TGGAAATAGAT-GGG] according to the following cycle conditions: 94 °C for 2 min followed by 15 cycles of 15 sec at 94 °C, 30 sec at 55 °C, and 2 min at 72 °C.

The 1^{st} PCR products were diluted by 2,000 times, and 1 µL of each diluted PCR product was subjected to a 20 µL Real-Time PCR reaction against sets of exon specific primers sets [Exon 4_forward(q): (5' → 3') GACCATGTTTTGCCCATTG and Exon 4_reverse(q): (5' → 3') GGCTCTTTTGAAGAAGACC for exon 4; Exon 5_forward(q): (5' → 3') GAAACAGAAGTA-CCTGTGC and Exon 5_reverse(q): (5' → 3') GTCATCCCTGCTTCATAAC for exon 5; and Exon 6_forward(q): (5' → 3') CGGAAGCTGAAGAAACTTG and Exon 6_reverse(q): (5' → 3') CACTTGACCACGTGTACAAG for exon 6]. The PCR reactions were monitored by SYBR Green (Takara, Japan). Cycle Conditions: 95 °C for 3 min followed by 40 cycles 5 sec at 95 °C, and 30 sec at 60°C.

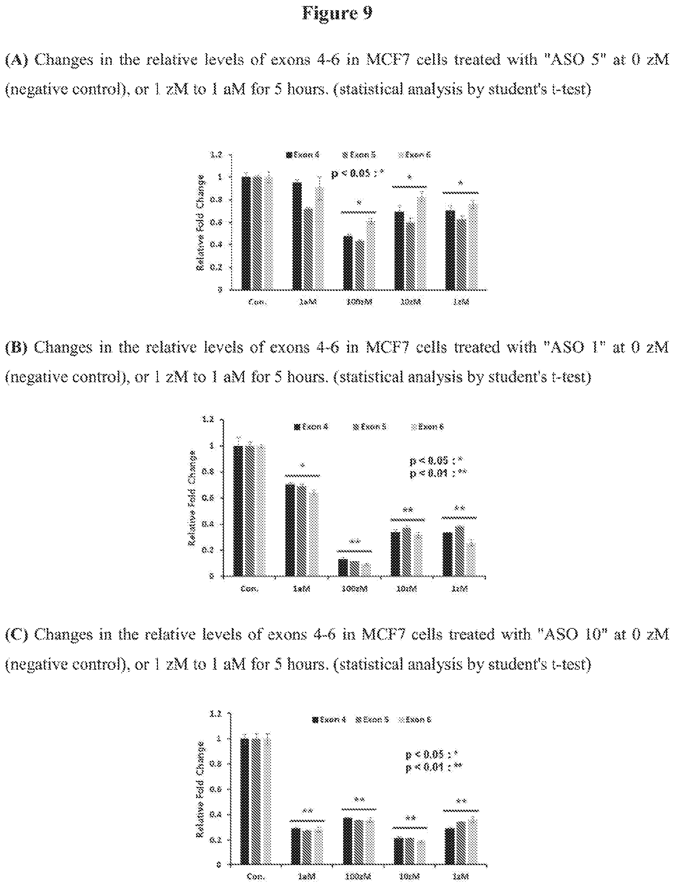

Figure 9(A) provides the qPCR data obtained therefrom. The relative expression level of exons 4-6 significantly decreased as the ASO concentration was increased from 0 zM to 100 zM. At 100 zM, the exon message levels decreased by ca 50 to 60%. At 1 aM, however, the exon message levels rebounded to near the levels of the negative control (no ASO treatment). The strange dose response pattern of the qPCR data could be due to a transcription upregulation by the "exon intron circular RNA (EIciRNA)" accumulated during the exon skipping with "ASO 5". [Nature Struc. Mol. Biol. vol 22(3), 256-264 (2015)]

### Example 3. qPCR Evaluation of AR mRNA Level in MCF Cells treated with "ASO 1".

Although "ASO 1" specified in Table 1 is a 13-mer antisense oligonucleotide originally designed to complementarily target the junction of "exon 5" and "exon 6" within the human AR mRNA. "ASO 1" has a 9-mer complementary overlap with the 9-mer sequence as marked "bold" and "underlined" in the 20-mer RNA sequence [(5' → 3') GC**CUUGCCUG** | **g**"uaag"gaaaa] spanning the junction of "exon 5" and "intron 5" within the human AR pre-mRNA. It is noted that the four single mismatches in intron 5 are marked as "uaag". Thus "ASO 1" may be regarded as an antisense oligonucleotide targeting the human AR pre-mRNA, although only with a 9-mer complementary overlap out of the 13-mer sequence.

"ASO 1" was evaluated for its ability to down-regulate the human AR mRNA by qPCR according to the protocol described in "Example 2".

Figure 9(B) provides the qPCR data obtained therefrom. The relative expression level of "exons 4-6" significantly decreased as the ASO concentration was increased from 0 zM to 100 zM. At 100 zM, the exon message levels decreased by more than 80%. At 1 aM, however, the exon message levels rebounded to ca 60% of the negative control (no ASO treatment). The strange dose response pattern of the qPCR data could be due to a transcription upregulation by the "exon intron circular RNA (EIciRNA)" accumulated during the exon skipping with "ASO 5". [Nature Struc. Mol. Biol. vol 22(3), 256-264 (2015)]

### Example 4. qPCR Evaluation of AR mRNA Level in MCF Cells treated with "ASO 10".

"ASO 10" specified in Table 1 is a 12-mer antisense oligonucleotide which has a 12-mer full complementary overlap with the 12-mer sequence as marked "bold" and "underlined" in the 20-mer pre-mRNA sequence [(5' → 3') GCC**UUGCCUG** | **guaag**gaaaa] spanning the junction of "exon 5" and "intron 5" of the human AR pre-mRNA.

"ASO 10" was evaluated for its ability to down-regulate the human AR mRNA (full-length) by qPCR according to the protocol described in "Example 2".

Figure 9(C) provides the qPCR data obtained therefrom. The relative expression level of "exons 4-6" significantly decreased by 60 ~ 80% in MCF7 cells treated with "ASO 10" at 1 zM to 1,000 zM.

### Example 5. Western Blot Analysis of AR Downregulation by "ASO 1 ".

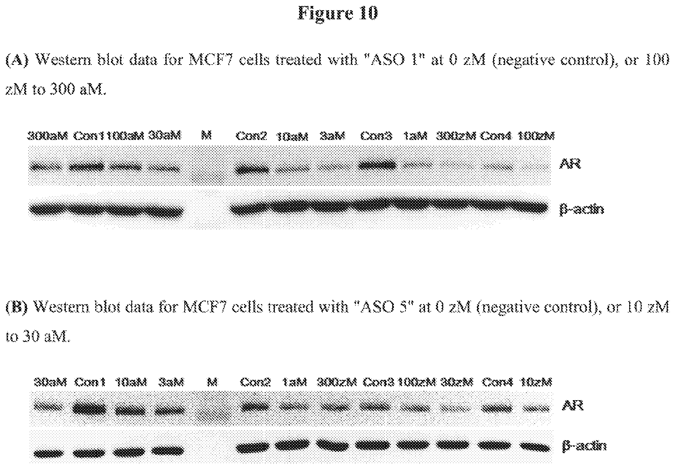

MCF7 cells were sub-cultured in 60 mm culture dish containing 5 ml culture medium, and treated with "ASO 1" at 0 zM (negative control), or 100 zM to 300 aM. 4 culture dishes were used for 4 negative controls. 48 hours later, cells were washed 2 times with cold PBS, and then subjected to lysis with 200 µL 1X cell lysis buffer (Cat. No. 9803, Cell Signaling Tech) supplemented with 1X protease inhibitor (Cat. No. P8340, Sigma). The lysates were collected in 1.5 ml e-tube. 200 µL of each lysate was mixed with 100 µL 3X sample buffer, and boiled for 5 min at 100 °C. 20 µL of each lysate (12 lysates in total). 4 negative controls and 8 ASO treatment samples) was subjected to electrophoretic separation on a 8% SDS-PAGE gel, and transferred onto a 0.2 µm PVDF membrane. The membrane was probed with anti-AR antibody (Cat. No. 5153, Cell Signaling Tech) and anti-β-actin antibody (Cat. No. sc4778, Santa Cruz). Figure 10(A) provides the AR Western blot data obtained therefrom. Multiple (negative) control samples were used to overcome technical artifacts of western blot procedures. Except for the AR band for (negative) "control 4", the AR band intensity of the lysates with ASO treatment was considerably weaker than that of the lysates without ASO treatment, which unequivocally indicates that "ASO 1" inhibits the expression of the full-length AR protein in MCF7 cells.

### Example 6. Western Blot Analysis of AR Downregulation by "ASO 5".

"ASO 5" was evaluated for its ability to inhibit AR protein expression at 10 zM to 30 aM in MCF7 cells according to the procedures described in "Example 5".

Figure 10(B) provides the AR Western blot data obtained with MCF7 cells treated with "ASO 5" at 0 zM (negative control, no ASO treatment), or 10 zM to 30 aM. Multiple (negative) control samples were used to overcome technical artifacts of western blot procedures. The AR band intensity of the lysates with ASO treatment was considerably weaker than that of the neighboring lysates without ASO treatment, which unequivocally indicates that "ASO 1" inhibits the expression of the full-length AR protein in MCF7 cells.

### Example 7. Hair Growth Promoted by Topical Administration of "ASO 1" in Mice.

"ASO 1" was evaluated for its ability to promote hair growth in C57BL/6 mice upon topical administration as follows. The target sequence of "ASO 1" in the human AR pre-mRNA is conserved in the mouse AR pre-mRNA. Thus the in vivo therapeutic findings in mice may be extrapolated to human cases without much ambiguity.

[Hair Removal and Grouping] In Day 0, 7 week old female C57BL/6 mice were anesthetized with zoletil/rompun, and the hair in the back was cut and removed with a clipper and wax, respectively. Mice with flawless (i.e. spotless) hair removal were selected and randomly assigned into three groups (7 animals per group).

[Topical Administration] The topical solutions of "ASO 1" were prepared by diluting a mother stock solution of "ASO 1" to 0.2 fM or 1 fM in aqueous 30%(v/v) ethanol supplemented with 3%(v/v) glycerin. About 100 µL of 0 (negative control), 0.2, or 1 fM "ASO 1" was topically administered in the back of each animal using a cotton ball in Days 3, 7, 10, and 14.

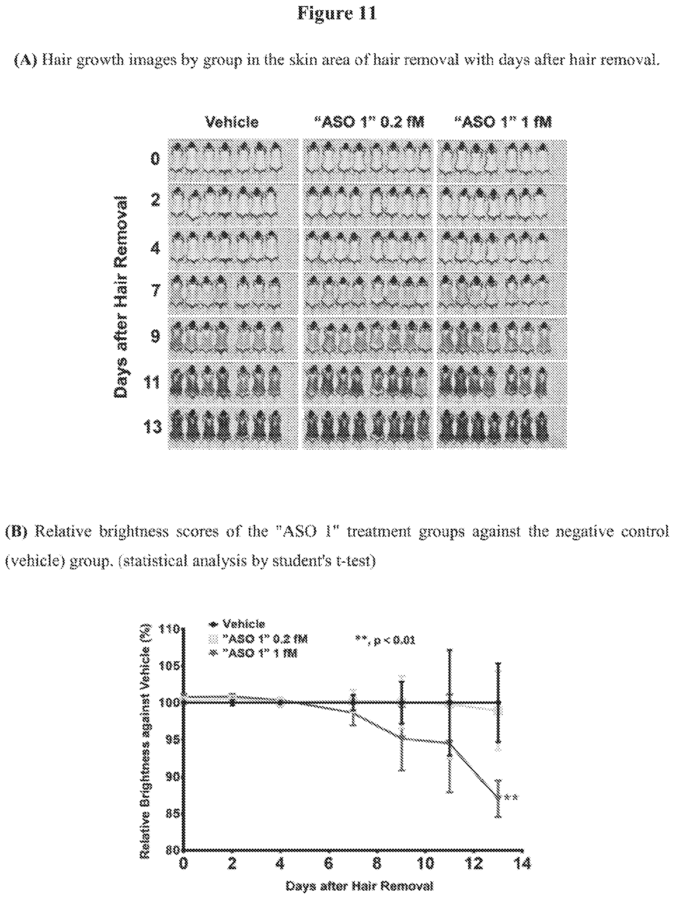

[Digital Image Scoring for Hair Growth] For scoring the hair growth, the animals were anesthetized and photographed by group as shown in Figure 11(A) using a digital camera at a fixed value of exposure time and illumination. The digital image for the area of the hair removal for each animal was selected and digitally scored for the average brightness over the selected area using "ImageJ" program. Lower brightness score is taken as faster hair growth. Brightness scores of individual animals were combined by group, and subjected to statistical analysis by student's t-test. Figure 11(B) summarizes the relative brightness scores of the ASO treated groups against the control group. The relative brightness score tended to decrease with days in the treatment groups. In Day 13, the 1 fM group was significantly lower in the brightness score than the non-treated group. Thus, "ASO 1" was concluded to promote hair growth upon topical administrations at 1 fM.

[Scoring by Hair Weight] In Day 21, the animals were anesthetized and the hair in the back was cut with a clipper. The hair samples from individual animals were combined by group, and weighed to evaluate the hair growth between Day 0 and Day 21. The average hair weight was 70.5 mg/animal for the control group, 90.8 mg/animal for the 0.2 fM treatment group, and 94.4 mg/animal for the 1 fM treatment group. Thus "ASO 1" was concluded to promote hair growth upon topical administrations at 0.2 fM as well as 1 fM.

[AR IHC of Skin Sample] Following the shaving in Day 21, the mice received a single topical administration of either vehicle or "ASO 1" according to the group. In Day 24, the skin of the area with hair removal was sampled for immunohistochemistry (IHC) analysis against androgen receptor. The skin samples were cryo-sectioned and subjected to immunostaining in series with a primary anti-AR antibody (Cat. No. sc-816, Santa Cruz) at 1:100 dilution, with a secondary anti-IgG (Cat No. BA-1100, Vector) at 1:200 dilution, and then with Dylight 594-steptavidin (Cat No. SA-5594, Vector, CA, USA) at 1:200 dilution for red fluoresence tagging. The IHC images were captured on an Olympus fluorescence microscope for changes in the AR expression level upon topical treatment with "ASO 1".

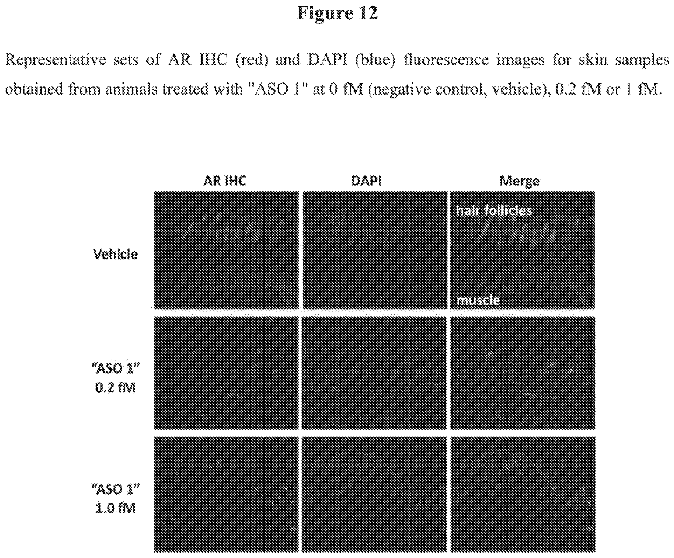

Figure 12 is a representative set of AR IHC images demonstrating that the AR expression in hair follicles was markedly inhibited in hair follicles upon the topical administrations of "ASO 1" at 0.2 fM or 1 fM. The DAPI staining images were provided to locate the hair follicles in the IHC images. It is interesting to note that AR expression decreased even in the muscle layer underneath the dermis upon the topical administrations of "ASO 1" at 0.2 fM or 1 fM. Thus "ASO 1" is readily delivered into the dermis as well as the muscle layer underneath the dermis upon topical administration, and potently inhibits the expression of AR.

### Example 8. Hair Growth Promoted by Topical Administration of "ASO 5" in Mice.

"ASO 5" was evaluated for its ability to promote hair growth in C57BL/6 mice upon topical administration as detailed below. The target sequence of "ASO 5" in the human AR pre-mRNA is conserved in the mouse AR pre-mRNA. Thus the in vivo therapeutic findings in mice may be extrapolated to human cases without much ambiguity.

[Hair Removal and Grouping] In Day 0, 7 week old female C57BL/6 mice were anesthetized with zoletil/rompun, and the hair in the back was cut and removed with a clipper and wax, respectively. Mice with flawless (i.e. spotless) hair removal were selected and randomly assigned into three groups (10 animals per group).

[Topical Administration] The topical solutions of "ASO 5" were prepared by diluting a mother stock solution of "ASO 5" to 1, 5, or 25 fM in aqueous 30%(v/v) ethanol supplemented with 3%(v/v) glycerin. About 100 µL of each ASO solution or vehicle (negative control) was topically administered to in the back of an animal using a cotton ball in Days 3, 7, 10, 14 and 21.

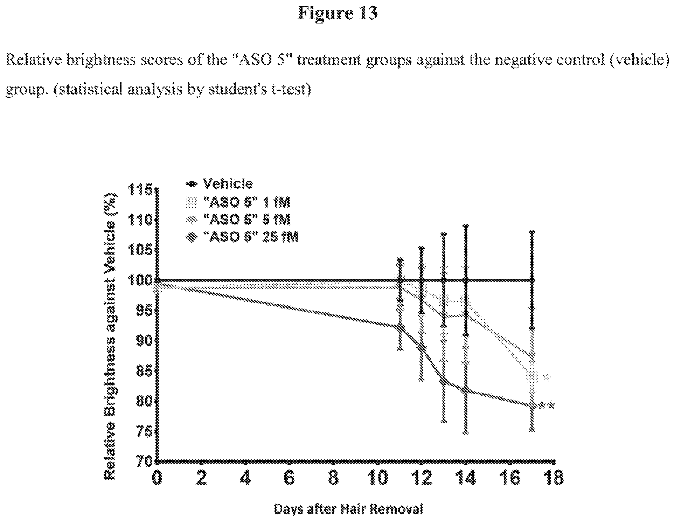

[Digital Image Scoring for Hair Growth] Figure 13 summarizes the relative brightness scores of the ASO treated groups against the negative control group. The relative brightness score tended to decrease with days in the ASO treatment groups. In Day 17, the treatment groups of 1 fM and 25 fM were significantly lower in the brightness score than the non-treated group. Thus, "ASO 5" was concluded to promote hair growth upon topical administrations at 1 to 25 fM.

[Scoring by Hair Weight] In Day 21, the animals were anesthetized and the hair in the back was cut and collected. The hair samples from individual animals were combined by group, and weighed to evaluate the hair growth between Day 0 and Day 21. The treatment groups yielded marked increases in the hair weight compared to the control group. The average hair weights of the 1 fM, 5 fM, and 25 fM groups were 293%, 306%, and 278% of the negative control group, respectively. Thus "ASO 5" was concluded to promote hair growth upon topical administrations at 1 to 25 fM.

Following the shaving in Day 21, the animals received a single topical administration of either vehicle or "ASO 5" at 1 fM, 5 fM, or 25 fM. In Day 53, the hair in the back was collected by shaving with a clipper to determine the total amount of hair growth between Days 21 and 53. The average hair weights of the 1 fM, 5 fM, and 25 fM groups were 1,630%, 1,450%, and 771% of the non-treated group, respectively. Thus "ASO 5" was concluded to promote hair growth upon topical administrations at 1 to 25 fM.

### Example 9. Hair Growth Promoted by Topical Administration of "ASO 10" in Mice.

"ASO 10" was evaluated for its ability to promote hair growth in C57BL/6 mice upon topical administration as detailed below. The target sequence of "ASO 10" in the human AR pre-mRNA is conserved in the mouse AR pre-mRNA. Thus the in vivo therapeutic findings in mice may be extrapolated to human cases without much ambiguity.

[Hair Removal and Grouping] In Day 0, 7 week old female C57BL/6 mice were anesthetized with zoletil/rompun, and the hair in the back was cut and removed with a clipper and wax, respectively. Mice with flawless (i.e. spotless) hair removal were selected and randomly assigned into three groups (9 animals per group).

[Topical Administration] The topical solutions of "ASO 10" were prepared by diluting a mother stock solution of "ASO 10" to 1, 5, or 25 fM in aqueous 30%(v/v) ethanol supplemented with 3%(v/v) glycerin. About 100 µL of each ASO solution or vehicle (negative control) was topically administered to in the back of an animal using a cotton ball in Day 2.

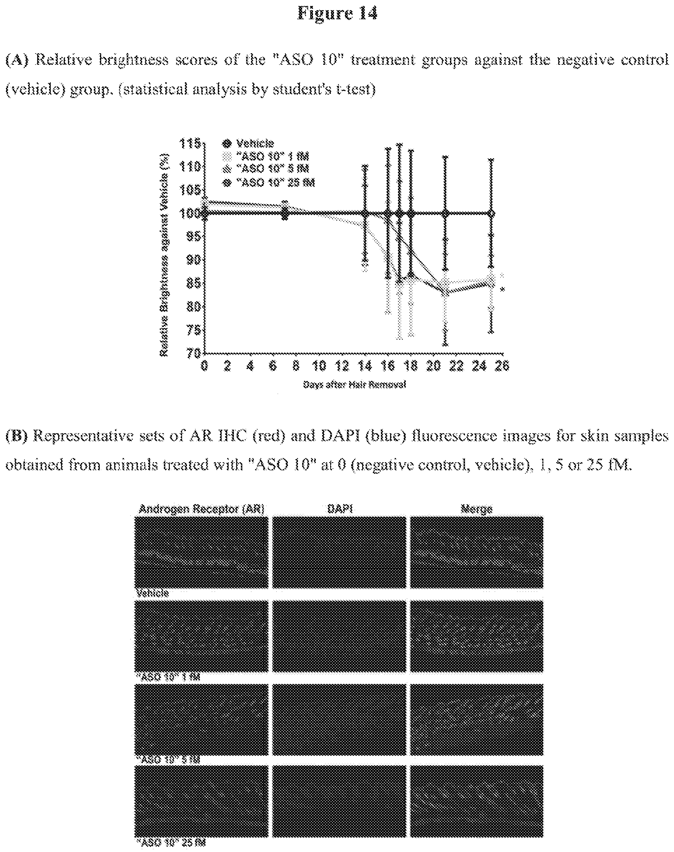

[Digital Image Scoring for Hair Growth] Figure 14(A) summarizes the relative brightness scores of the ASO treated groups against the control group. The relative brightness score tended to decrease with days in the treatment groups. In Day 27, the treatment groups of 1 fM and 5 fM were significantly lower in the brightness score than the non-treatment group. Thus, "ASO 10" was concluded to promote hair growth upon topical administrations at 1 to 5 fM.

[Scoring by Hair Weight] In Day 27, the animals were anesthetized and the hair in the back was cut and collected. The hair samples from individual animals were combined by group, and weighed to evaluate the hair growth between Day 0 and Day 27. The treatment groups yielded modest increases in the hair weight compared to the control group. The average hair weights of the 1 fM, 5 fM, and 25 fM groups were 115%, 114%, and 119% of the non-treated group, respectively. Thus "ASO 10" was concluded to promote hair growth upon topical administrations at 1 to 25 fM.

[AR Immunohistochemistry of Skin Samples] After the hair cut in Day 27, The animals topically received further either vehicle or "ASO 10" at 1, 5, or 25 fM in Days 27 and 29. Skin samples of the back were collected in Day 30 for immunohistochemical analysis against androgen receptor. The skin samples were subjected to immunostaining against androgen receptor as described in "Example 7". IHC images were captured on a Zeiss slide scanner. Figure 14(B) is a representative set of AR IHC images. It is interesting to note that the number of hair follicles increased markedly in the ASO treatment groups compared to the negative control group. It would be difficult to clearly state that the AR expression in hair follicles decreased in the treatment groups due to the marked increases in the number of hair follicles in the treatment groups. Nevertheless, the AR expression in the muscle layer underneath the dermis markedly decreased in the treatment groups compared to the control group. The most notable decrease was observed with the 5 fM treatment group. Taken together the IHC findings in the animals treated with "ASO 10", "ASO 10" promotes hair growth by inhibiting AR expression to increase the number of hair follicles at all the tested doses, most notably at 5 fM.

### Example 10. qPCR Evaluation of AR mRNA Level by TaqMan Assay in MCF7 Cells treated with "ASO 10".

"ASO 10" was evaluated for its ability to down-regulate the human AR mRNA by qPCR adopting a TaqMan probe.

MCF7 cells sub-cultured in 5 mL culture medium in 60 mm culture dish, and treated or with "ASO 10" at 0 zM (negative control) to 1 aM (2 culture dishes per each concentration). 24 hours later, total RNA was extracted by "MiniBEST Universal RNA Extraction Kit" according to the manufacturer's instructions (Cat. No. 9767, Takara).

400 ng of RNA template were used to synthesize cDNA for a 20 µL reverse transcription reaction using One-Step RT-PCR kit (Invitrogen) against a set of exon specific primers of [exon 3_forward: (5' —> 3') TGGGTGTCACTATGGAGC ; and exon 9_reverse: (5' → 3') GGGTGT- GGAAATAGATGGG] according to the following cycle conditions: 50 °C for 30 min and 94 °C for 2 min, followed by 15 cycles of 30 sec at 94°C, 30 sec at 50 °C, and 1 min at 72 °C.

The cDNA solutions were diluted by 50 times, and 1 µL of each diluted PCR product was subjected to a 20 µL Real-Time PCR reaction against a set of exon specific primers of [exon 4_forward: (5' —> 3') TTGTCCATCTTGTCGTCTT; and exon 5_reverse: (5' —> 3') CCTCTC-CTTCCTCCTGTA] according to the following cycle conditions: 95 °C for 3 min followed by 40 cycles 15 sec at 95 °C, and 30 sec at 60 °C. The qPCR reaction was monitored with a TaqMan probe of [(5' ----+ 3') TTTCTTCAG-ZEN-CTTCCGGGCTC-3IABkFQ].

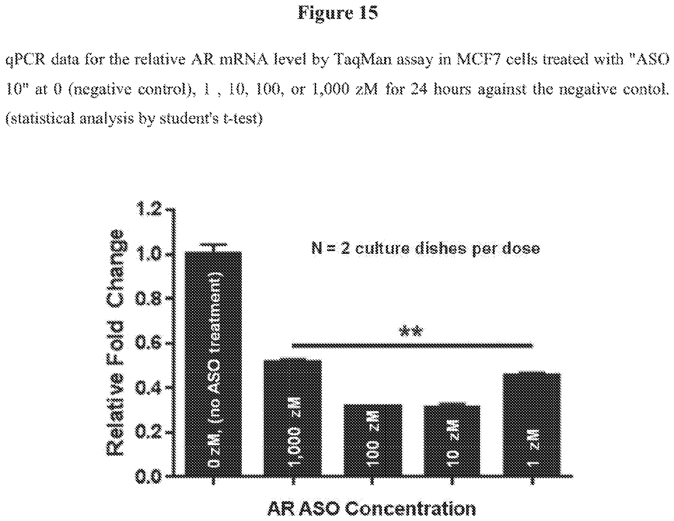

Figure 15 provides the qPCR data obtained therefrom. The relative expression level of exons 4-6 significantly decreased by ca 50 to 70% in MCF7 cells treated with "ASO 10" at 1 zM to 1 aM.

### Example 11. Down-regulation of AR Expression by IHC in Mice Subcutaneously Administered with "ASO 10".

"ASO 10" was evaluated for its ability to inhibit AR expression in mice as follows. 12 weeks old male C57BL/6 mice were randomly assigned to 3 groups of negative control (no ASO treatment), 0.01 pmole/Kg "ASO 10" and 0.1 pmole/Kg "ASO 10". (3 animals per group) Mice subcutaneously received either vehicle or ASO dissolved in vehicle (PBS) 2X per week for 4 weeks according to the dosing group. Three days post the final dosing, the animals were anesthetized with zoletil/rompun and subjected to tissue or organ sampling for AR IHC by paraffin blolck.

The AR protein was probed in series with a primary antibody (Cat. No. sc-816, Santa Cruz) at 1: 100 dilution, a secondary anti-rabbit IgG (Cat. No. BA-1100, VECTOR) at 1:200 dilution, and Dylight 594-Streptavidin (Cat. No. SA-5594, VECTOR) at 1:200. Nucleus was stained with DAPI. IHC fluorescence images were captured on a Zeiss slide scanner.

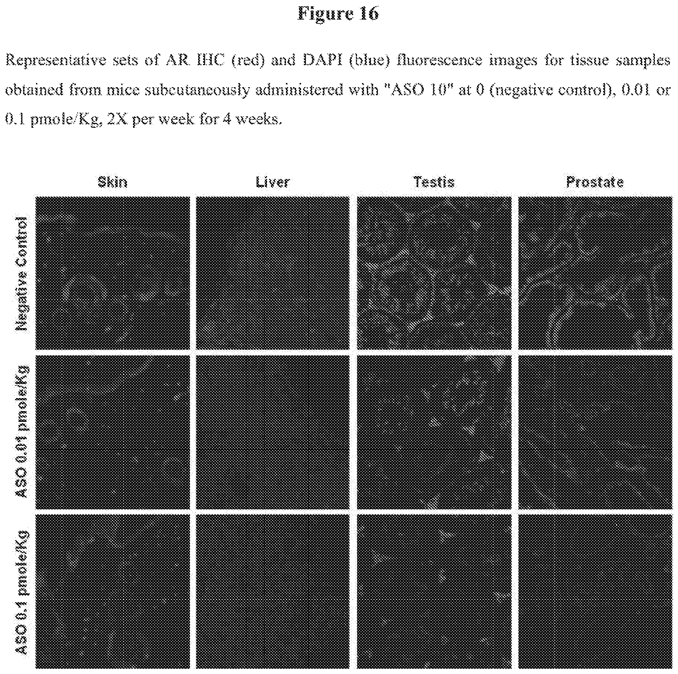

Figure 16 provides AR IHC images (red) obtained with tissues known to abundantly express AR protein. It is noted that the IHC images are provided as colocalized with DAPI (blue). Upon chronic subcutaneous exposure to "ASO 10", AR expression markedly decreased in epidermis distal to the injection site, the liver, testis and prostate as the ASO dose was increased from 0.01 pmole/Kg to 0.1 pmole/Kg. Thus "ASO 10" unequivocally inhibits AR protein expression in mice upon systemic exposure

## Claims

1. A peptide nucleic acid derivative represented by **Formula I,** or a pharmaceutically acceptable salt thereof: 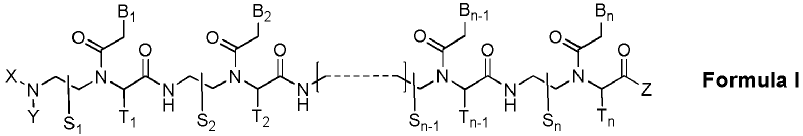 wherein,
n is an integer between 11 and 21;
the compound of **Formula I** possesses at least a 9-mer complementary overlap with a 17-mer RNA sequence of [(5' —> 3') CCUUGCCUGGUAAGGAA] within the human androgen receptor pre-mRNA;
S₁, S₂, ..., Sₙ₋₁, Sₙ, T₁, T₂, ..., Tₙ₋₁, and Tₙ independently represent deuterido, hydrido, substituted or non-substituted alkyl, or substituted or non-substituted aryl radical;
X and Y independently represent hydrido [H], formyl [H-C(=O)-], aminocarbonyl [NH₂-C(=O)-], substituted or non-substituted alkyl, substituted or non-substituted aryl, substituted or non-substituted alkylacyl, substituted or non-substituted arylacyl, substituted or non-substituted alkyloxycarbonyl, substituted or non-substituted aryloxycarbonyl, substituted or non-substituted alkylaminocarbonyl, substituted or non-substituted arylaminocarbonyl, substituted or non-substituted alkylsulfonyl, or substituted or non-substituted arylsulfonyl radical;
Z represents hydrido, hydroxy, substituted or non-substituted alkyloxy, substituted or non-substituted aryloxy, substituted or non-substituted amino, substituted or non-substituted alkyl, or substituted or non-substituted aryl radical;
B₁, B₂, ..., Bₙ₋₁, and Bₙ are independently selected from natural nucleobases including adenine, thymine, guanine, cytosine and uracil, and unnatural nucleobases; and
at least four of B₁, B₂, ..., Bₙ₋₁, and Bₙ are independently selected from unnatural nucleobases with a substituted or non-substituted amino radical covalently linked to the nucleobase moiety.

2. The peptide nucleic acid derivative according to claim 1, or a pharmaceutical salt thereof:
wherein,
n is an integer between 11 and 15;
the compound of **Formula I** possesses at least a 11-mer complementary overlap with the 17-mer RNA sequence of [(5' ----+ 3') CCUUGCCUGGUAAGGAA] within the human AR pre-mRNA;
the compound of **Formula I** is fully complementary to a pre-mRNA sequence within the human AR pre-mRNA;
S₁, S₂, ..., Sₙ₋₁, Sₙ, T₁, T₂, ..., Tₙ₋₁, and Tₙ are hydrido radical;
X is hydrido radical;
Y represents substituted or non-substituted alkylacyl, or substituted or non-substituted alkyloxycarbonyl radical;
Z represents substituted or non-substituted amino radical;
B₁, B₂, ..., Bₙ₋₁, and Bₙ are independently selected from adenine, thymine, guanine, cytosine, and unnatural nucleobases;
at least five of B₁, B₂, ..., Bₙ₋₁, and Bₙ are independently selected from unnatural nucleobases represented by **Formula II, Formula III,** or **Formula IV;** 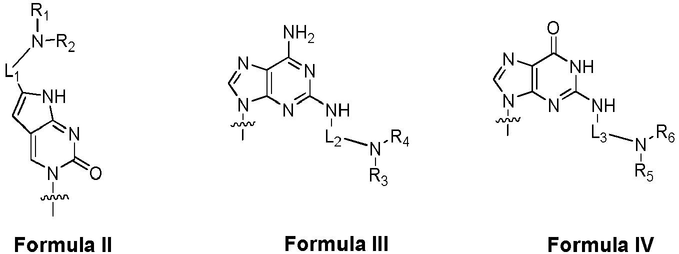 wherein
R₁, R₂, R₃, R₄, R₅, and R₆ are hydrido radical;
L₁ represents -(CH₂)₂-O-(CH₂)₂-, -CH₂-O-(CH₂)₂-, -CH₂-O-(CH₂)₃-, -CH₂-O-(CH₂)₄-, or -CH₂-O-(CH₂)₅- with the right end being directly linked to the basic amino group; and,
L₂ and L₃ are independently selected from -(CH₂)₂-O-(CH₂)₂-, -(CH₂)₃-O-(CH₂)₂-, - (CH₂)₂-O-(CH₂)₃-, -(CH₂)₂-, -(CH₂)₃-, -(CH₂)₄-, -(CH₂)₅-, -(CH₂)₆-, -(CH₂)₇-, and -(CH₂)₈- with the right end being directly linked to the basic amino group.

3. The peptide nucleic acid derivative according to claim 1, which is selected from the group of peptide nucleic acid derivatives provided below, or a pharmaceutically acceptable salt thereof:
(N ----+ C) Fmoc-GA(5)A-GC(1O2)C-A(5)GG-C(1O2)AA(5)-G-NH₂;
(N ----+ C) Fethoc-GA(5)A-GC(1O2)C-A(5)GG-C(1O2)AA(5)-G-NH₂;
(N ----+ C) Ac-GA(5)A-GC(1O2)C-A(5)GG-C(1O2)AA(5)-G-NH₂;
(N ----+ C) Benzoyl-GA(5)A-GC(1O2)C-A(2O2)GG-C(1O2)AA(5)-G-NH₂;
(N ----+ C) Piv-GA(5)A-GC(1O2)C-A(5)GG-C(1O2)AA(5)-G-NH₂;
(N ----+ C) Methyl-GA(5)A-GC(1O2)C-A(5)GG-C(1O2)AA(5)-G-NH₂;
(N ----+ C) n-Propyl-GA(5)A-GC(1O2)C-A(5)GG-C(1O2)AA(5)-G-NH₂;
(N ----+ C) Fmoc-Lys-GA(5)A-GC(1O2)C-A(5)GG-C(1O2)AA(5)-G-NH₂;
(N ----+ C) Fmoc-Lys-GA(5)A-GC(1O2)C-A(5)GG-C(1O2)AA(5)-G-Lys-NH₂
(N ----+ C) Fmoc-GA(5)A-GC(1O2)C-A(5)GG-C(1O2)AA(5)-G-Lys-NH₂;
(N ----+ C) Fmoc-Gly-GA(5)A-GC(1O2)C-A(5)GG-C(1O2)AA(5)-G-NH₂;
(N ----+ C) Fmoc-Lys-Gly-GA(5)A-GC(1O2)C-A(5)GG-C(1O2)AA(5)-G-Lys-NH₂;
(N ----+ C) Fmoc-Val-Gly-GA(5)A-GC(1O2)C-A(5)GG-C(1O2)AA(5)-G-NH₂;
(N ----+ C) Fmoc-GA(6)A-GC(102)C-A(6)GG-C(102)AA(6)-G-NH₂;
(N ----+ C) Fmoc-G(6)AA(5)-GC(103)C-A(7)GG(5)-CA(5)A-G-NH₂;
(N ----+ C) Fmoc-GA(5)A-GC(2O2)C-A(6)GG-C(1O5)AA(6)-G-NH₂;
(N ----+ C) Fmoc-TG(6)C(1O5)-GGA(6)-AG(6)C-CA(6)G-GC(1O2)A-A(6)GG(6)-NH₂;
(N ----+ C) Fethoc-C(1O2)TT-A(5)CC-A(5)GG-C(1O2)AA(5)-G-NH₂;
(N ----+ C) Fethoc-C(1O2)TT-A(5)CC-A(5)GG-C(1O2)AA(5)-G-Lys-Lys-NH₂;
(N ----+ C) Fethoc-C(102)TT-A(6)CC-A(5)GG-C(103)AA(5)-G-Val-Lys-NH₂;
(N ----+ C) Ac-C(1O2)TT-A(5)CC-A(5)GG-C(1O2)TA(5)-G-NH₂;
(N ----+ C) Piv-C(102)TT-A(5)CC-A(5)GG-C(102)AA(5)-G-NH₂;
(N ----+ C) H-C(1O2)TT-A(5)CC-A(5)GG-C(1O2)AA(5)-G-NH₂;
(N ----+ C) H-CTT-A(5)C(103)C-A(5)G(3)G-C(102)AA(5)-G-NH₂;
(N ----+ C) n-Propyl-C(1O2)TT-A(5)CC-A(5)GG-C(1O2)AA(5)-G-NH₂;
(N ----+ C) n-Propyl-CTT-A(5)C(2O2)C-A(3)G(2O3)G-C(lO2)AA(5)-G-NH₂;
(N ----+ C) p-Toluenesulfonyl-CTT-A(5)C(102)C-A(8)G(5)G-C(102)AA(5)-G-NH₂;
(N ----+ C) Benzoyl-Lys-Val-C(1O2)TT-A(5)CC-A(5)GG-C(1O2)AA(5)-G-NH₂;
(N ----+ C) Benzoyl-CTT-A(5)C(1O5)C-A(5)G(2O2)G-C(1O2)AA(5)-G-NH₂;
(N ----+ C) Fethoc-Lys-Leu-CTT-A(5)C(1O2)C-A(2O2)GG-C(1O2)AA(5)-G-Lys-NH₂;
(N ----+ C) Fethoc-C(1O2)TT-A(5)CC-A(5)GG-C(1O5)AA(5)-G-NH₂;
(N ----+ C) Fethoc-C(1O2)TT-A(5)CC-A(5)GT-C(1O2)AA(5)-G-NH₂;
(N ----+ C) Fethoc-C(1O2)TT-A(5)CC-A(5)GG-C(1O2)TA(5)-G-NH₂;
(N ----+ C) Fethoc-C(1O2)TT-A(5)CC-A(5)GG-C(1O2)A(5)A-G-NH₂;
(N ----+ C) Fethoc-C(1O2)TT-A(5)CC-AG(5)G-C(1O2)AA(5)-G-NH₂;
(N ----+ C) Fethoc-C(1O2)TT-A(5)CC-A(7)GG-C(1O2)AA(3)-G-NH₂;
(N ----+ C) Fethoc-CTT-A(5)C(1O2)C-A(5)GT-C(1O2)TA(5)-G-NH₂;
(N ----+ C) Fethoc-CTT-A(5)C(1O2)C-A(5)GT-C(1O2)TA(5)-G-Arg-NH₂;
(N ----+ C) Fethoc-TC(102)C-TTA(6)-CCA(6)-GGC(102)-AA(6)G-G(6)-NH₂;
(N ----+ C) Fethoc-TC(102)C-TTA(5)-CCA(5)-GGC(102)-AA(5)G-G(6)-NH₂;
(N ----+ C) Fethoc-GA(5)T-AC(102)C-A(5)GG(6)-CAA(5)-G-NH₂;
(N ----+ C) Fethoc-TA(5)C-CAG(6)-GC(1O2)A-A(5)GG(6)-C-NH₂;
(N ----+ C) Fethoc-C(102)TT-A(5)CC-A(5)GG(6)-CA(5)A-NH₂;
(N ----+ C) Benzyl-C(1O2)TT-A(2O2)CC-A(5)GG(6)-CA(5)A-NH₂;
(N ----+ C) Phenyl-C(1O2)TT-A(5)CC-A(5)GG(6)-CA(5)A-NH₂;
(N ----+ C) Fethoc-C(1O2)TT-A(5)CC-A(7)GG(5)-CA(5)A-NH₂;
(N ----+ C) Fethoc-C(1O2)TT-A(5)CC-A(6)GG(2O2)-CA(5)A-NH₂;
(N ----+ C) Fethoc-C(1O2)TT-A(5)CC-A(5)GG(6)-CA(5)A-Val-Lys-NH₂;
(N ----+ C) Piv-C(102)TT-A(5)CC-A(5)GG(6)-CA(5)A-NH₂;
(N ----+ C) Fmoc-Lys-Val-C(1O2)TT-A(5)CC-A(5)GG(6)-CA(5)A-NH₂;
(N ----+ C) Ac-C(1O2)TT-A(5)CC-A(5)GG(6)-CA(5)A-NH₂;
(N ----+ C) H-C(1O2)TT-A(5)CC-A(5)GG(6)-CA(5)A-Lys-NH₂;
(N ----+ C) Piv-Arg-C(1O2)TT-A(5)CC-A(5)GG(6)-CA(5)A-Lys-NH₂;
(N ----+ C) N-Phenyl-N-methyl-CTT-A(5)C(1O2)C-A(5)GG-C(1O2)AA(5)-G-Lys-NH₂;
(N ----+ C) [N-(2-Phenylethyl)amino]carbonyl-CTT-A(5)C(1O2)C-A(4)G(5)G-C(1O2)AA(5)-G-NH₂;
(N ----+ C) Benzoyl-Leu-C(1O2)TT-A(5)CC-A(5)GG(6)-CA(5)A-Lys-NH₂;
(N ----+ C) Fethoc-C(1O3)TT-A(5)CC-A(5)GG(5)-CA(5)A-NH₂;
(N ----+ C) Fethoc-C(102)TT-A(6)CC-A(6)GG(6)-CA(6)A-NH₂;
(N ----+ C) Fethoc-TTT-TCC(102)-TTA(6)-CCA(6)-GG(6)C-A(6)A-NH₂;
(N ----+ C) Fethoc-TTT-TCC(1O2)-TTA(6)-CC(1O3)A(6)-G-Lys-NH₂;
(N ----+ C) Fethoc-TC(2O2)C-TTA(6)-CCA(6)-GG(6)C-A(6)A-NH₂;
(N → C) Me-Gly-TC(2O2)C-TTA(6)-CCA(6)-GG(6)C-A(6)A-NH₂;
(N ----+ C) Fethoc-Lys-TTT-TCC(1O2)-TTA(6)-CCA(6)-GG(6)C-NH₂; and
(N ----+ C) Fethoc-Arg-TCC(1O2)-TTA(5)-CCA(6)-GG(5)C-Lys-NH₂;
wherein
A, G, T, and C are PNA monomers with a natural nucleobase of adenine, guanine, thymine, and cytosine, respectively;
C(pOq), A(p), A(pOq), G(p), and G(pOq) are PNA monomers with an unnatural nucleobase represented by **Formula VI, Formula VII, Formula VIII, Formula IX,** and **Formula X,** respectively; 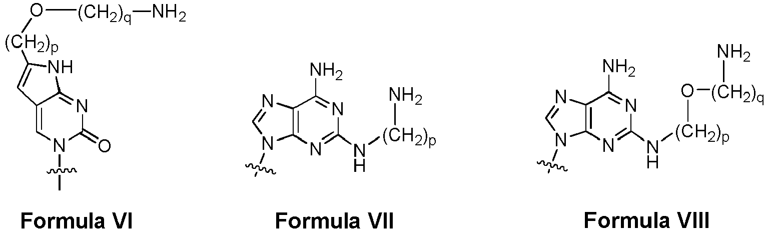 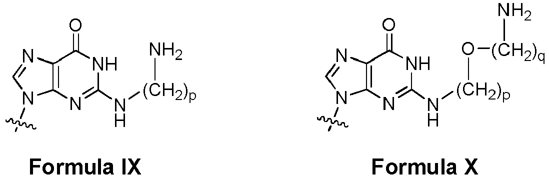 wherein,
p and q are integers; and
the abbreviations for the N- and C-terminus substituents are as specifically described as follows: "Fmoc-" is the abbreviation for "[(9-fluorenyl)methyloxy]carbonyl-"; "Fethoc-" for "[2-(9-fluorenyl)ethyl-1-oxy]carbonyl"; "Ac-" for "acetyl-"; "Benzoyl-" for "benzenecabonyl-"; "Piv-" for "pivalyl-"; "Methyl-" for "methyl-"; "n-Propyl-" for "1-(n-propyl)-"; "H-" for "hydrido-" group; "p-Toluenesulfonyl" for "(4-methylbenzene)-1-sulfonyl- "; "-Lys-" for amino acid residue "lysine"; "-Val-" for amino acid residue "valine"; "-Leu-" for amino acid residue "leucine"; "-Arg-" for amino acid residue "arginine"; "-Gly-" for amino acid residue "glycine"; "[N-(2-Phenylethyl)amino]carbonyl-" for "[N-1-(2-phenylethyl)amino]carbonyl-"; "Benzyl-" for "1-(phenyl)methyl-"; "Phenyl-" for "phenyl-"; "Me-" for "methyl-"; and "-NH₂" for non-substituted "-amino" group.

4. The peptide nucleic acid derivative according to claim 1, which is selected from the group of compounds provided below, or a pharmaceutically acceptable salt thereof:
(N → C) Fmoc-GA(5)A-GC(1O2)C-A(5)GG-C(1O2)AA(5)-G-NH₂;
(N → C) Fethoc-GA(5)A-GC(1O2)C-A(5)GG-C(1O2)AA(5)-G-NH₂;
(N → C) Fmoc-GA(6)A-GC(1O2)C-A(6)GG-C(1O2)AA(6)-G-NH₂;
(N → C) Fmoc-G(6)AA(5)-GC(1O3)C-A(7)GG(5)-CA(5)A-G-NH₂;
(N → C) Fmoc-GA(5)A-GC(2O2)C-A(6)GG-C(1O5)AA(6)-G-NH₂;
(N → C) Fethoc-TG(6)C(1O2)-GGA(6)-AG(6)C-CA(6)G-GC(1O2)A-A(6)GG(6)-NH₂;
(N → C) Fethoc-C(102)TT-A(5)CC-A(5)GG-C(102)AA(5)-G-NH₂;
(N → C) Fethoc-C(102)TT-A(6)CC-A(5)GG-C(103)AA(5)-G-Val-Lys-NH₂;
(N → C) Ac-C(102)TT-A(5)CC-A(5)GG-C(102)TA(5)-G-NH₂;
(N → C) Piv-C(102)TT-A(5)CC-A(5)GG-C(102)AA(5)-G-NH₂;
(N → C) H-CTT-A(5)C(103)C-A(5)G(3)G-C(102)AA(5)-G-NH₂;
(N → C) n-Propyl-C(102)TT-A(5)CC-A(5)GG-C(102)AA(5)-G-NH₂;
(N → C) n-Propyl-CTT-A(5)C(202)C-A(3)G(203)G-C(102)AA(5)-G-NH₂;
(N → C) p-Toluenesulfonyl-CTT-A(5)C(102)C-A(8)G(5)G-C(102)AA(5)-G-NH₂;
(N → C) Benzoyl-CTT-A(5)C(105)C-A(5)G(202)G-C(102)AA(5)-G-NH₂;
(N → C) Fethoc-Lys-Leu-CTT-A(5)C(1O2)C-A(2O2)GG-C(1O2)AA(5)-G-Lys-NH₂;
(N → C) Fethoc-C(102)TT-A(5)CC-A(5)GG-C(105)AA(5)-G-NH₂;
(N → C) Fethoc-C(102)TT-A(5)CC-A(7)GG-C(102)AA(3)-G-NH₂;
(N → C) Fethoc-CTT-A(5)C(102)C-A(5)GT-C(102)TA(5)-G-NH₂;
(N → C) Fethoc-TC(102)C-TTA(6)-CCA(6)-GGC(102)-AA(6)G-G(6)-NH₂;
(N → C) Fethoc-TC(102)C-TTA(5)-CCA(5)-GGC(102)-AA(5)G-G(6)-NH₂;
(N → C) Fethoc-GA(5)T-AC(102)C-A(5)GG(6)-CAA(5)-G-NH₂;
(N → C) Fethoc-TA(5)C-CAG(6)-GC(1O2)A-A(5)GG(6)-C-NH₂;
(N → C) Fethoc-C(102)TT-A(5)CC-A(5)GG(6)-CA(5)A-NH₂;
(N → C) Fethoc-C(102)TT-A(5)CC-A(7)GG(5)-CA(5)A-NH₂;
(N → C) Fethoc-C(102)TT-A(5)CC-A(6)GG(202)-CA(5)A-NH₂;
(N → C) Piv-C(1O2)TT-A(5)CC-A(5)GG(6)-CA(5)A-H₂;;
(N → C) Ac-C(102)TT-A(5)CC-A(5)GG(6)-CA(5)A-NH₂;
(N → C) N-Phenyl-N-methyl-CTT-A(5)C(102)C-A(5)GG-C(102)AA(5)-G-Lys-NH₂;
(N → C) [N-(2-Phenylethyl)amino]carbonyl-CTT-A(5)C(102)C-A(4)G(5)G-C(102)AA(5)-G-NH₂;
(N → C) Fethoc-C(102)TT-A(6)CC-A(6)GG(6)-CA(6)A-NH₂;
(N → C) Fethoc-TTT-TCC(102)-TTA(6)-CCA(6)-GG(6)C-A(6)A-NH₂;
(N → C) Fethoc-TTT-TCC(1O2)-TTA(6)-CC(1O3)A(6)-G-Lys-NH₂;and
(N → C) Fethoc-TC(2O2)C-TTA(6)-CCA(6)-GG(6)C-A(6)A-NH₂.

5. A peptide nucleic acid derivative according to claim 1, selected from the group consisting of: (N → C) Fethoc-C(1O2)TT-A(5)CC-A(5)GG-C(1O2)AA(5)-G-NH₂, (N → C) Fethoc-TC(1O2)C-TTA(6)-CCA(6)-GGC(1O2)-AA(6)G-G(6)-NH₂, (N → C) Fethoc-TC(1O2)C-TTA(5)-CCA(5)-GGC(1O2)-AA(5)G-G(6)-NH₂, (N --7 C) Fethoc-TA(5)C-CAG(6)-GC(1O2)A-A(5)GG(6)-C-NH₂, (N --7 C) Fethoc-C(102)TT-A(5)CC-A(5)GG(6)-CA(5)A-NH₂, (N --7 C) Fethoc-C(1O2)TT-A(5)CC-A(6)GG(6)-CA(5)A-NH₂, (N --7 C) Ac-C(1O2)TT-A(5)CC-A(5)GG(6)-CA(5)A-NH₂, or (N --7 C) Fethoc-C(102)TT-A(6)CC-A(6)GG(6)-CA(6)A-NH₂, or a pharmaceutically acceptable salt thereof.

6. A peptide nucleic acid derivative according to any one of claims 1 to 5, for use in the treatment of a dermatological indication involving androgenic activity.

7. A peptide nucleic acid derivative of any one of claims 1 to 5, for use in the treatment of a dermatological condition involving androgenic activity.

8. A peptide nucleic acid derivative of any one of claims 1 to 5, for use in the treatment of androgenic alopecia.

## Patentansprüche

1. Peptid-Nukleinsäurederivat, dargestellt durch **Formel I,** oder ein pharmazeutisch unbedenkliches Salz davon: 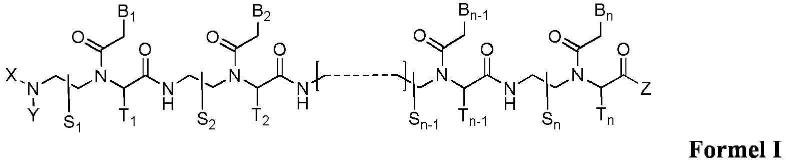 wobei
n eine ganze Zahl zwischen 11 und 21 ist;
die Verbindung von **Formel I** zumindest eine komplementäre 9-mer-Überlappung mit einer 17-mer-RNA-Sequenz von [(5' → 3') CCUUGCCUGGUAAGGAA] innerhalb der menschlichen Androgenrezeptor-prä-mRNA besitzt;
S₁, S₂, ..., Sₙ₋₁, Sₙ, T₁, T₂, ..., Tₙ₋₁ und Tₙ unabhängig Deuterido-, Hydrid-, substituiertes oder nicht substituiertes Alkyl- oder substituiertes oder nicht substituiertes Aryl-Radikal darstellen;
X und Y unabhängig Hydrid- [H], Formyl- [H-C(=O)-], Aminocarbonyl- [NH₂-C(=O)-], substituiertes oder nicht substituiertes Alkyl-, substituiertes oder nicht substituiertes Aryl-, substituiertes oder nicht substituiertes Alkylacyl-, substituiertes oder nicht substituiertes Arylacyl-, substituiertes oder nicht substituiertes Alkyloxycarbonyl-, substituiertes oder nicht substituiertes Aryloxycarbonyl-, substituiertes oder nicht substituiertes Alkylaminocarbonyl-, substituiertes oder nicht substituiertes Arylaminocarbonyl-, substituiertes oder nicht substituiertes Alkylsulfonyl- oder substituiertes oder nicht substituiertes Arylsulfonyl-Radikal darstellen;
Z Hydrid-, Hydroxy-, substituiertes oder nicht substituiertes Alkyloxy-, substituiertes oder nicht substituiertes Aryloxy-, substituiertes oder nicht substituiertes Amino-, substituiertes oder nicht substituiertes Alkyl- oder substituiertes oder nicht substituiertes Aryl-Radikal darstellt;
B₁, B₂, ..., Bₙ₋₁ und Bₙ unabhängig aus natürlichen Nukleinbasen, die Adenin, Thymin, Guanin, Cytosin und Uracil beinhalten, und unnatürlichen Nukleinbasen ausgewählt sind; und
zumindest vier von B₁, B₂, ..., Bₙ₋₁ und Bₙ unabhängig aus unnatürlichen Nukleinbasen mit einem substituierten oder nicht substituierten Amino-Radikal ausgewählt sind, das kovalent mit der Nukleinbaseneinheit verbunden ist.

2. Peptid-Nukleinsäurederivat gemäß Anspruch 1 oder ein pharmazeutisches Salz davon:
wobei
n eine ganze Zahl zwischen 11 und 15 ist;
die Verbindung von **Formel I** zumindest eine komplementäre 11-mer-Überlappung mit der 17-mer-RNA-Sequenz von [(5' → 3') CCUUGCCUGGUAAGGAA] innerhalb der menschlichen AR-prä-mRNA besitzt;
die Verbindung von **Formel I** zu einer Prä-mRNA-Sequenz innerhalb der humanen AR-prä-mRNA vollständig komplementär ist;
S₁, S₂, ..., Sₙ₋₁, Sₙ, T₁, T₂, ..., Tₙ₋₁ und Tₙ Hydrid-Radikale sind;
X Hydrid-Radikal ist;
Y substituiertes oder nicht substituiertes Alkylacyl oder substituiertes oder nicht substituiertes Alkyloxycarbonyl-Radikal darstellt;
Z substituiertes oder nicht substituiertes Amino-Radikal darstellt;
B₁, B₂, Bₙ₋₁ und Bₙ unabhängig aus Adenin, Thymin, Guanin, Cytosin und unnatürlichen Nukleinbasen ausgewählt sind;
zumindest fünf von B₁, B₂, ..., Bₙ₋₁ und Bₙ unabhängig aus unnatürlichen Nukleinbasen ausgewählt sind, die durch **Formel II, Formel III** oder **Formel IV** dargestellt sind; 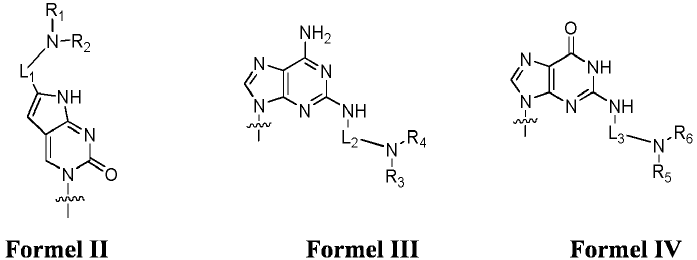 wobei
R₁, R₂, R₃, R₄, R₅ und R₆ Hydrid-Radikale sind;
L₁ -(CH₂)₂-O-(CH₂)₂-, -CH₂-O-(CH₂)₂-, -CH₂-O-(CH₂)₃-, -CH₂-O-(CH₂)₄- oder -CH₂-O-(CH₂)₅- darstellt, wobei das rechte Ende direkt mit der basischen Aminogruppe verbunden ist; und
L₂ und L₃ unabhängig ausgewählt sind aus -(CH₂)₂-O-(CH₂)₂-, -(CH₂)₃-O-(CH₂)₂-, - (CH₂)₂-O-(CH₂)₃-, -(CH₂)₂-, -(CH₂)₃-, -(CH₂)₄-, -(CH₂)₅-, -(CH₂)₆-, -(CH₂)₇-, and -(CH₂)₈-, wobei das rechte Ende direkt mit der basischen Aminogruppe verbunden ist.

3. Peptid-Nukleinsäurederivat gemäß Anspruch 1, das ausgewählt ist aus der nachfolgend aufgezählten Gruppe von Peptid-Nukleinsäurederivaten oder einem pharmazeutisch unbedenklichen Salz davon:
(N → C) Fmoc-GA(5)A-GC(1O2)C-A(5)GG-C(1O2)AA(5)-G-NH₂;
(N → C) Fethoc-GA(5)A-GC(1O2)C-A(5)GG-C(1O2)AA(5)-G-NH₂;
(N → C) Ac-GA(5)A-GC(1O2)C-A(5)GG-C(1O2)AA(5)-G-NH₂;
(N → C) Benzoyl-GA(5)A-GC(1O2)C-A(2O2)GG-C(1O2)AA(5)-G-NH₂;
(N → C) Piv-GA(5)A-GC(1O2)C-A(5)GG-C(1O2)AA(5)-G-NH₂;
(N → C) Methyl-GA(5)A-GC(1O2)C-A(5)GG-C(1O2)AA(5)-G-NH₂;
(N → C) n-Propyl-GA(5)A-GC(1O2)C-A(5)GG-C(1O2)AA(5)-G-NH₂;
(N → C) Fmoc-Lys-GA(5)A-GC(1O2)C-A(5)GG-C(1O2)AA(5)-G-NH₂;
(N → C) Fmoc-Lys-GA(5)A-GC(1O2)C-A(5)GG-C(1O2)AA(5)-G-Lys-NH₂
(N → C) Fmoc-GA(5)A-GC(1O2)C-A(5)GG-C(1O2)AA(5)-G-Lys-NH₂;
(N → C) Fmoc-Gly-GA(5)A-GC(1O2)C-A(5)GG-C(1O2)AA(5)-G-NH₂;
(N → C) Fmoc-Lys-Gly-GA(5)A-GC(1O2)C-A(5)GG-C(1O2)AA(5)-G-Lys-NH₂;
(N → C) Fmoc-Val-Gly-GA(5)A-GC(1O2)C-A(5)GG-C(1O2)AA(5)-G-NH₂;
(N → C) Fmoc-GA(6)A-GC(1O2)C-A(6)GG-C(1O2)AA(6)-G-NH₂;
(N → C) Fmoc-G(6)AA(5)-GC(1O3)C-A(7)GG(5)-CA(5)A-G-NH₂;
(N → C) Fmoc-GA(5)A-GC(2O2)C-A(6)GG-C(1O5)AA(6)-G-NH₂;
(N → C) Fmoc-TG(6)C(1O5)-GGA(6)-AG(6)C-CA(6)G-GC(1O2)A-A(6)GG(6)- NH₂;
(N → C) Fethoc-C(1O2)TT-A(5)CC-A(5)GG-C(1O2)AA(5)-G-NH₂;
(N → C) Fethoc-C(1O2)TT-A(5)CC-A(5)GG-C(1O2)AA(5)-G-Lys-Lys-NH₂;
(N → C) Fethoc-C(1O2)TT-A(6)CC-A(5)GG-C(1O3)AA(5)-G-Val-Lys-NH₂;
(N → C) Ac-C(1O2)TT-A(5)CC-A(5)GG-C(1O2)TA(5)-G-NH₂;
(N → C) Piv-C(1O2)TT-A(5)CC-A(5)GG-C(1O2)AA(5)-G-NH₂;
(N → C) H-C(1O2)TT-A(5)CC-A(5)GG-C(1O2)AA(5)-G-NH₂;
(N → C) H-CTT-A(5)C(1O3)C-A(5)G(3)G-C(1O2)AA(5)-G-NH₂;
(N → C) n-Propyl-C(1O2)TT-A(5)CC-A(5)GG-C(1O2)AA(5)-G-NH₂;
(N → C) n-Propyl-CTT-A(5)C(2O2)C-A(3)G(2O3)G-C(1O2)AA(5)-G-NH₂;
(N → C) p-Toluolsulfonyl-CTT-A(5)C(1O2)C-A(8)G(5)G-C(1O2)AA(5)-G-NH₂;
(N → C) Benzoyl-Lys-Val-C(1O2)TT-A(5)CC-A(5)GG-C(1O2)AA(5)-G-NH₂;
(N → C) Benzoyl-CTT-A(5)C(1O5)C-A(5)G(2O2)G-C(1O2)AA(5)-G-NH₂;
(N → C) Fethoc-Lys-Leu-CTT-A(5)C(1O2)C-A(2O2)GG-C(1O2)AA(5)-G-Lys-NH₂;
(N → C) Fethoc-C(1O2)TT-A(5)CC-A(5)GG-C(1O5)AA(5)-G-NH₂;
(N → C) Fethoc-C(1O2)TT-A(5)CC-A(5)GT-C(1O2)AA(5)-G-NH₂;
(N → C) Fethoc-C(1O2)TT-A(5)CC-A(5)GG-C(1O2)TA(5)-G-NH₂;
(N → C) Fethoc-C(1O2)TT-A(5)CC-A(5)GG-C(1O2)A(5)A-G-NH₂;
(N → C) Fethoc-C(1O2)TT-A(5)CC-AG(5)G-C(1O2)AA(5)-G-NH₂;
(N → C) Fethoc-C(1O2)TT-A(5)CC-A(7)GG-C(1O2)AA(3)-G-NH₂;
(N → C) Fethoc-CTT-A(5)C(1O2)C-A(5)GT-C(1O2)TA(5)-G-NH₂;
(N → C) Fethoc-CTT-A(5)C(1O2)C-A(5)GT-C(1O2)TA(5)-G-Arg-NH₂;
(N → C) Fethoc-TC(1O2)C-TTA(6)-CCA(6)-GGC(1O2)-AA(6)G-G(6)-NH₂;
(N → C) Fethoc-TC(1O2)C-TTA(5)-CCA(5)-GGC(1O2)-AA(5)G-G(6)-NH₂;
(N → C) Fethoc-GA(5)T-AC(1O2)C-A(5)GG(6)-CAA(5)-G-NH₂;
(N → C) Fethoc-TA(5)C-CAG(6)-GC(1O2)A-A(5)GG(6)-C-NH₂;
(N → C) Fethoc-C(1O2)TT-A(5)CC-A(5)GG(6)-CA(5)A-NH₂;
(N → C) Benzyl-C(1O2)TT-A(2O2)CC-A(5)GG(6)-CA(5)A-NH₂;
(N → C) Phenyl-C(1O2)TT-A(5)CC-A(5)GG(6)-CA(5)A-NH₂;
(N → C) Fethoc-C(1O2)TT-A(5)CC-A(7)GG(5)-CA(5)A-NH₂;
(N → C) Fethoc-C(1O2)TT-A(5)CC-A(6)GG(2O2)-CA(5)A-NH₂;
(N → C) Fethoc-C(1O2)TT-A(5)CC-A(5)GG(6)-CA(5)A-Val-Lys-NH₂;
(N → C) Piv-C(1O2)TT-A(5)CC-A(5)GG(6)-CA(5)A-NH₂;
(N → C) Fmoc-Lys-Val-C(1O2)TT-A(5)CC-A(5)GG(6)-CA(5)A-NH₂;
(N → C) Ac-C(1O2)TT-A(5)CC-A(5)GG(6)-CA(5)A-NH₂;
(N → C) H-C(1O2)TT-A(5)CC-A(5)GG(6)-CA(5)A-Lys-NH₂;
(N → C) Piv-Arg-C(1O2)TT-A(5)CC-A(5)GG(6)-CA(5)A-Lys-NH₂;
(N → C) N-Phenyl-N-methyl-CTT-A(5)C(1O2)C-A(5)GG-C(1O2)AA(5)-G-Lys-NH₂;
(N → C) [N-(2-Phenylethyl)amino]carbonyl-CTT-A(5)C(102)C-A(4)G(5)G-C(1O2)AA(5)-G-NH₂;
(N → C) Benzoyl-Leu-C(1O2)TT-A(5)CC-A(5)GG(6)-CA(5)A-Lys-NH₂;
(N -> C) Fethoc-C(1O3)TT-A(5)CC-A(5)GG(5)-CA(5)A-NH₂;
(N → C) Fethoc-C(1O2)TT-A(6)CC-A(6)GG(6)-CA(6)A-NH₂;
(N → C) Fethoc-TTT-TCC(1O2)-TTA(6)-CCA(6)-GG(6)C-A(6)A-NH₂;
(N → C) Fethoc-TTT-TCC(1O2)-TTA(6)-CC(1O3)A(6)-G-Lys-NH₂;
(N → C) Fethoc-TC(2O2)C-TTA(6)-CCA(6)-GG(6)C-A(6)A-NH₂;
(N → C) Me-Gly-TC(2O2)C-TTA(6)-CCA(6)-GG(6)C-A(6)A-NH₂;
(N → C) Fethoc-Lys-TTT-TCC(1O2)-TTA(6)-CCA(6)-GG(6)C-NH₂; und
(N → C) Fethoc-Arg-TCC(1O2)-TTA(5)-CCA(6)-GG(5)C-Lys-NH₂;
wobei
A, G, T und C jeweils PNA-Monomere mit einer natürlichen Nukleinbase von Adenin, Guanin, Thymin und Cytosin sind;
C(pOq), A(p), A(pOq), G(p) und G(pOq) PNA-Monomere mit einer unnatürlichen Nukleinbase sind, jeweils dargestellt durch **Formel VI, Formel VII, Formel VIII, Formel IX** und **Formel X;** 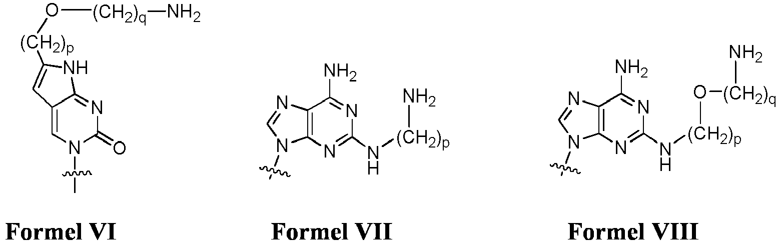 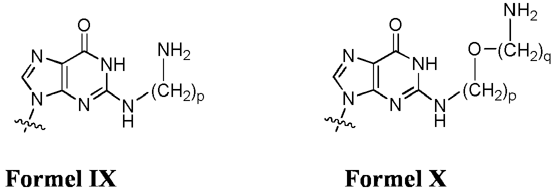 wobei
p und q ganze Zahlen sind; und
die Abkürzungen für die Substituenten am N- und C-Terminus wie folgt spezifisch beschrieben sind: "Fmoc-" ist die Abkürzung für "[(9-Fluorenyl)methyloxy]carbonyl-"; "Fethoc-" für "[2-(9-Fluorenyl)ethyl-1-oxy]carbonyl"; "Ac-" für "Acetyl-"; "Benzoyl-" für "Benzolcabonyl-"; "Piv-" für "Pivalyl-"; "Methyl-" für "Methyl-"; "n-Propyl-" für "1-(n-Propyl)-"; "H-" für "Hydrid-"Gruppe; "p-Toluolsulfonyl" für "(4-Methylbenzol)-1-sulfonyl-"; "-Lys-" für Aminosäurerest "Lysin"; "-Val-" für Aminosäurerest "Valin"; "-Leu-" für Aminosäurerest "Leucin"; "-Arg-" für Aminosäurerest "Arginin"; "-Gly-" für Aminosäurerest "Glycin"; "[N-(2-Phenylethyl)amino]carbonyl-" für "[N-1-(2-Phenylethyl)amino]carbonyl-"; "Benzyl-" für "1-(Phenyl)methyl-"; "Phenyl-" für "Phenyl-"; "Me-" für "Methyl-"; und "-NH₂" für nicht substituierte "-Amino"-Gruppe.

4. Peptid-Nukleinsäurederivat gemäß Anspruch 1, das ausgewählt ist aus der nachfolgend aufgezählten Gruppe von Verbindungen oder einem pharmazeutisch unbedenklichen Salz davon ausgewählt ist:
(N → C) Fmoc-GA(5)A-GC(1O2)C-A(5)GG-C(1O2)AA(5)-G-NH₂;
(N → C) Fethoc-GA(5)A-GC(1O2)C-A(5)GG-C(1O2)AA(5)-G-NH₂;
(N → C) Fmoc-GA(6)A-GC(1O2)C-A(6)GG-C(1O2)AA(6)-G-NH₂;
(N → C) Fmoc-G(6)AA(5)-GC(1O3)C-A(7)GG(5)-CA(5)A-G-NH₂;
(N → C) Fmoc-GA(5)A-GC(2O2)C-A(6)GG-C(1O5)AA(6)-G-NH₂;
(N → C) Fethoc-TG(6)C(1O2)-GGA(6)-AG(6)C-CA(6)G-GC(1O2)A-A(6)GG(6)-NH₂;
(N → C) Fethoc-C(1O2)TT-A(5)CC-A(5)GG-C(1O2)AA(5)-G-NH₂;
(N → C) Fethoc-C(1O2)TT-A(6)CC-A(5)GG-C(1O3)AA(5)-G-Val-Lys-NH₂;
(N → C) Ac-C(1O2)TT-A(5)CC-A(5)GG-C(1O2)TA(5)-G-NH₂;
(N → C) Piv-C(1O2)TT-A(5)CC-A(5)GG-C(1O2)AA(5)-G-NH₂;
(N → C) H-CTT-A(5)C(1O3)C-A(5)G(3)G-C(1O2)AA(5)-G-NH₂;
(N → C) n-Propyl-C(1O2)TT-A(5)CC-A(5)GG-C(1O2)AA(5)-G-NH₂;
(N → C) n-Propyl-CTT-A(5)C(2O2)C-A(3)G(2O3)G-C(1O2)AA(5)-G-NH₂;
(N → C) p-Toluolsulfonyl-CTT-A(5)C(1O2)C-A(8)G(5)G-C(1O2)AA(5)-G-NH₂;
(N → C) Benzoyl-CTT-A(5)C(1O5)C-A(5)G(2O2)G-C(1O2)AA(5)-G-NH₂;
(N → C) Fethoc-Lys-Leu-CTT-A(5)C(1O2)C-A(2O2)GG-C(1O2)AA(5)-G-Lys-NH₂;
(N → C) Fethoc-C(1O2)TT-A(5)CC-A(5)GG-C(1O5)AA(5)-G-NH₂;
(N → C) Fethoc-C(1O2)TT-A(5)CC-A(7)GG-C(1O2)AA(3)-G-NH₂;
(N → C) Fethoc-CTT-A(5)C(1O2)C-A(5)GT-C(1O2)TA(5)-G-NH₂;
(N → C) Fethoc-TC(1O2)C-TTA(6)-CCA(6)-GGC(1O2)-AA(6)G-G(6)-NH₂;
(N → C) Fethoc-TC(1O2)C-TTA(5)-CCA(5)-GGC(1O2)-AA(5)G-G(6)-NH₂;
(N → C) Fethoc-GA(5)T-AC(1O2)C-A(5)GG(6)-CAA(5)-G-NH₂;
(N → C) Fethoc-TA(5)C-CAG(6)-GC(1O2)A-A(5)GG(6)-C-NH₂;
(N → C) Fethoc-C(1O2)TT-A(5)CC-A(5)GG(6)-CA(5)A-NH₂;
(N → C) Fethoc-C(1O2)TT-A(5)CC-A(7)GG(5)-CA(5)A-NH₂;
(N → C) Fethoc-C(1O2)TT-A(5)CC-A(6)GG(2O2)-CA(5)A-NH₂;
(N → C) Piv-C(102)TT-A(5)CC-A(5)GG(6)-CA(5)A-NH₂;;
(N → C) Ac-C(1O2)TT-A(5)CC-A(5)GG(6)-CA(5)A-NH₂;
(N → C) N-Phenyl-N-methyl-CTT-A(5)C(1O2)C-A(5)GG-C(1O2)AA(5)-G-Lys-NH₂;
(N → C) [N-(2-Phenylethyl)amino]carbonyl-CTT-A(5)C(102)C-A(4)G(5)G-C(1O2)AA(5)-G-NH₂;
(N → C) Fethoc-C(1O2)TT-A(6)CC-A(6)GG(6)-CA(6)A-NH₂;
(N → C) Fethoc-TTT-TCC(1O2)-TTA(6)-CCA(6)-GG(6)C-A(6)A-NH₂;
(N → C) Fethoc-TTT-TCC(1O2)-TTA(6)-CC(1O3)A(6)-G-Lys-NH₂; und
(N → C) Fethoc-TC(2O2)C-TTA(6)-CCA(6)-GG(6)C-A(6)A-NH₂,

5. Peptid-Nukleinsäurederivat gemäß Anspruch 1, ausgewählt aus der Gruppe bestehend aus: (N -> C) Fethoc-C(1O2)TT-A(5)CC-A(5)GG-C(1O2)AA(5)-G-NH₂, (N -> C) Fethoc-TC(1O2)C-TTA(6)-CCA(6)-GGC(1O2)-AA(6)G-G(6)-NH₂, (N -> C) Fethoc- TC(102)C-TTA(5)-CCA(5)-GGC(1O2)-AA(5)G-G(6)-NH₂, (N → C) Fethoc-TA(5)C- CAG(6)-GC(102)A-A(5)GG(6)-C-NH₂, (N → C) Fethoc-C(102)TT-A(5)CC-A(5)GG(6)- CA(5)A-NH₂, (N → C) Fethoc-C(1O2)TT-A(5)CC-A(6)GG(6)-CA(5)A-NH₂, (N → C) Ac- C(102)TT-A(5)CC-A(5)GG(6)-CA(5)A-NH₂ oder (N → C) Fethoc-C(102)TT-A(6)CC- A(6)GG(6)-CA(6)A-NH₂ oder einem pharmazeutisch unbedenklichen Salz davon.

6. Peptid-Nukleinsäurederivat gemäß einem der Ansprüche 1 bis 5 zur Verwendung bei der Behandlung einer dermatologischen Indikation mit androgener Aktivität.

7. Peptid-Nukleinsäurederivat nach einem der Ansprüche 1 bis 5 zur Verwendung bei der Behandlung einer dermatologischen Erkrankung mit androgener Aktivität.

8. Peptid-Nukleinsäurederivat nach einem der Ansprüche 1 bis 5 zur Verwendung bei der Behandlung von androgener Alopezie.

## Revendications

1. Dérivé d'acide nucléique peptidique représenté par la formule I, ou sel pharmaceutiquement acceptable de celui-ci : 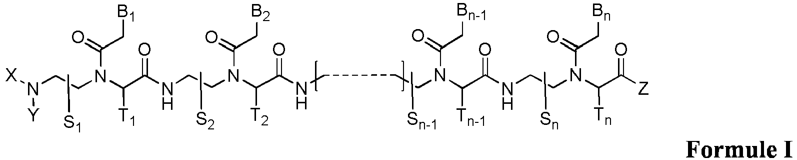 dans laquelle,
n est un entier compris entre 11 et 21 ;
le composé de **formule** I possède au moins un chevauchement complémentaire 9-mère avec une séquence d'ARN 17-mère de [(5' → 3') CCUUGCCUGGUAAGGAA] au sein du pré-ARNm de récepteur d'androgènes humain ;
S₁, S₂, ..., Sₙ₋₁, Sₙ, T₁, T₂, ..., Tₙ₋₁ et Tₙ représentent indépendamment un radical deutérido, hydrido, alkyle substitué ou non substitué, ou aryle substitué ou non substitué ;
X et Y représentent indépendamment un radical hydrido [H], formyle [H-C(=O)-], aminocarbonyle [NH₂-C(=O)-], alkyle substitué ou non substitué, aryle substitué ou non substitué, alkylacyle substitué ou non substitué, arylacyle substitué ou non substitué, alkyloxycarbonyle substitué ou non substitué, aryloxycarbonyle substitué ou non substitué, alkylaminocarbonyle substitué ou non substitué, arylaminocarbonyle substitué ou non substitué, alkylsulfonyle substitué ou non substitué, ou arylsulfonyle substitué ou non substitué ;
Z représente un radical hydrido, hydroxy, alkyloxy substitué ou non substitué, aryloxy substitué ou non substitué, amino substitué ou non substitué, alkyle substitué ou non substitué, ou aryle substitué ou non substitué ;
B₁, B₂, ..., Bₙ₋₁ et Bₙ sont indépendamment choisis parmi les nucléobases naturelles comprenant l'adénine, la thymine, la guanine, la cytosine et l'uracile, et les nucléobases non naturelles ; et
au moins quatre des groupes B₁, B₂, ..., Bₙ₋₁ et Bₙ sont indépendamment choisis parmi les nucléobases non naturelles avec un radical amino substitué ou non substitué lié par covalence au groupement nucléobase.

2. Dérivé d'acide nucléique peptidique selon la revendication 1, ou sel pharmaceutiquement acceptable de celui-ci :
n étant un entier compris entre 11 et 15 ;
ledit composé de **formule I** possédant au moins un chevauchement complémentaire 11-mère avec une séquence d'ARN 17-mère de [(5' → 3') CCUUGCCUGGUAAGGAA] au sein du pré-ARNm d'AR humain ;
ledit composé de **formule I** étant pleinement complémentaire à une séquence de pré-ARNm au sein du pré-ARNm d'AR humain ;
S₁, S₂, ..., Sₙ₋₁, Sₙ, T₁, T₂, ..., Tₙ₋₁ et Tₙ représentant un radical hydrido ;
X représentant un radical hydrido ;
Y représentant un radical alkylacyle substitué ou non substitué, ou alkyloxycarbonyle substitué ou non substitué ;
Z représentant un radical amino substitué ou non substitué ;
B₁, B₂, ..., Bₙ₋₁ et Bₙ étant indépendamment choisis parmi l'adénine, la thymine, la guanine, la cytosine, et les nucléobases non naturelles ;
au moins cinq des groupes B₁, B₂, ..., Bₙ₋₁ et Bₙ étant indépendamment choisis parmi les nucléobases non naturelles représentées par la **formule II, la formule III ou la formule IV ;** 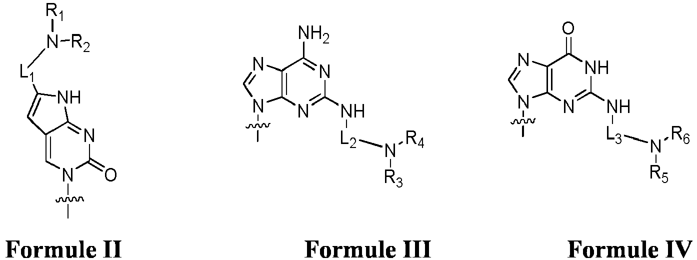 dans lesquelles
R₁, R₂, R₃, R₄, R₅ et R₆ sont un radical hydrido ;
Li représente un groupe -(CH₂)₂-O-(CH₂)₂-, -CH₂-O-(CH₂)₂-, -CH₂-O-(CH₂)₃-, -CH₂-O-(CH₂)₄- ou -CH₂-O-(CH₂)₅-, l'extrémité droite étant directement liée au groupe amino basique ; et,
L₂ et L₃ sont indépendamment choisis parmi les groupes -(CH₂)₂-O-(CH₂)₂-, -(CH₂)₃-O-(CH₂)₂-, -(CH₂)₂-O-(CH₂)₃-, -(CH₂)₂-, -(CH₂)₃-, -(CH₂)₄-, -(CH₂)₅-, -(CH₂)₆-, -(CH₂)₇- et -(CH₂)₈-, l'extrémité droite étant directement liée au groupe amino basique.

3. Dérivé d'acide nucléique peptidique selon la revendication 1, qui est choisi dans le groupe des dérivés d'acide nucléique peptidique fournis ci-après, ou sel pharmaceutiquement acceptable de celui-ci :
(N → C) Fmoc-GA(5)A-GC(1O2)C-A(5)GG-C(1O2)AA(5)-G-NH₂ ;
(N → C) Fethoc-GA(5)A-GC(1O2)C-A(5)GG-C(1O2)AA(5)-G-NH₂ ;
(N → C) Ac-GA(5)A-GC(1O2)C-A(5)GG-C(1O2)AA(5)-G-NH₂ ;
(N -> C) Benzoyl-GA(5)A-GC(1O2)C-A(2O2)GG-C(1O2)AA(5)-G-NH₂ ;
(N → C) Piv-GA(5)A-GC(1O2)C-A(5)GG-C(1O2)AA(5)-G-NH₂ ;
(N → C) Méthyl-GA(5)A-GC(1O2)C-A(5)GG-C(1O2)AA(5)-G-NH₂ ;
(N → C) n-Propyl-GA(5)A-GC(1O2)C-A(5)GG-C(1O2)AA(5)-G-NH₂ ;
(N → C) Fmoc-Lys-GA(5)A-GC(1O2)C-A(5)GG-C(1O2)AA(5)-G-NH₂ ;
(N → C) Fmoc-Lys-GA(5)A-GC(1O2)C-A(5)GG-C(1O2)AA(5)-G-Lys-NH₂ ;
(N → C) Fmoc-GA(5)A-GC(1O2)C-A(5)GG-C(1O2)AA(5)-G-Lys-NH₂ ;
(N → C) Fmoc-Gly-GA(5)A-GC(1O2)C-A(5)GG-C(1O2)AA(5)-G-NH₂ ;
(N → C) Fmoc-Lys-Gly-GA(5)A-GC(1O2)C-A(5)GG-C(1O2)AA(5)-G-Lys-NH₂ ;
(N → C) Fmoc-Val-Gly-GA(5)A-GC(1O2)C-A(5)GG-C(1O2)AA(5)-G-NH₂ ;
(N → C) Fmoc-GA(6)A-GC(1O2)C-A(6)GG-C(1O2)AA(6)-G-NH₂ ;
(N → C) Fmoc-G(6)AA(5)-GC(1O3)C-A(7)GG(5)-CA(5)A-G-NH₂ ;
(N → C) Fmoc-GA(5)A-GC(2O2)C-A(6)GG-C(1O5)AA(6)-G-NH₂ ;
(N → C) Fmoc-TG(6)C(1O5)-GGA(6)-AG(6)C-CA(6)G-GC(1O2)A-A(6)GG(6)-NH₂ ;
(N → C) Fethoc-C(1O2)TT-A(5)CC-A(5)GG-C(1O2)AA(5)-G-NH₂ ;
(N → C) Fethoc-C(1O2)TT-A(5)CC-A(5)GG-C(1O2)AA(5)-G-Lys-Lys-NH₂ ;
(N → C) Fethoc-C(1O2)TT-A(6)CC-A(5)GG-C(1O3)AA(5)-G-Val-Lys-NH₂ ;
(N → C) Ac-C(1O2)TT-A(5)CC-A(5)GG-C(1O2)TA(5)-G-NH₂ ;
(N → C) Piv-C(1O2)TT-A(5)CC-A(5)GG-C(1O2)AA(5)-G-NH₂ ;
(N → C) H-C(1O2)TT-A(5)CC-A(5)GG-C(1O2)AA(5)-G-NH₂ ;
(N → C) H-CTT-A(5)C(1O3)C-A(5)G(3)G-C(1O2)AA(5)-G-NH₂ ;
(N → C) n-Propyl-C(1O2)TT-A(5)CC-A(5)GG-C(1O2)AA(5)-G-NH₂ ;
(N → C) n-Propyl-CTT-A(5)C(2O2)C-A(3)G(2O3)G-C(1O2)AA(5)-G-NH₂ ;
(N → C) p-Toluènesulfonyl-CTT-A(5)C(1O2)C-A(8)G(5)G-C(1O2)AA(5)-G-NH₂ ;
(N → C) Benzoyl-Lys-Val-C(1O2)TT-A(5)CC-A(5)GG-C(1O2)AA(5)-G-NH₂ ;
(N → C) Benzoyl-CTT-A(5)C(1O5)C-A(5)G(2O2)G-C(1O2)AA(5)-G-NH₂ ;
(N → C) Fethoc-Lys-Leu-CTT-A(5)C(1O2)C-A(2O2)GG-C(1O2)AA(5)-G-Lys-NH₂ ;
(N → C) Fethoc-C(1O2)TT-A(5)CC-A(5)GG-C(1O5)AA(5)-G-NH₂ ;
(N → C) Fethoc-C(1O2)TT-A(5)CC-A(5)GT-C(1O2)AA(5)-G-NH₂ ;
(N → C) Fethoc-C(1O2)TT-A(5)CC-A(5)GG-C(1O2)TA(5)-G-NH₂ ;
(N → C) Fethoc-C(1O2)TT-A(5)CC-A(5)GG-C(1O2)A(5)A-G-NH₂ ;
(N → C) Fethoc-C(1O2)TT-A(5)CC-AG(5)G-C(1O2)AA(5)-G-NH₂ ;
(N → C) Fethoc-C(1O2)TT-A(5)CC-A(7)GG-C(1O2)AA(3)-G-NH₂ ;
(N → C) Fethoc-CTT-A(5)C(1O2)C-A(5)GT-C(1O2)TA(5)-G-NH₂ ;
(N → C) Fethoc-CTT-A(5)C(1O2)C-A(5)GT-C(1O2)TA(5)-G-Arg-NH₂ ;
(N → C) Fethoc-TC(1O2)C-TTA(6)-CCA(6)-GGC(1O2)-AA(6)G-G(6)-NH₂ ;
(N → C) Fethoc-TC(1O2)C-TTA(5)-CCA(5)-GGC(1O2)-AA(5)G-G(6)-NH₂ ;
(N → C) Fethoc-GA(5)T-AC(1O2)C-A(5)GG(6)-CAA(5)-G-NH₂ ;
(N → C) Fethoc-TA(5)C-CAG(6)-GC(1O2)A-A(5)GG(6)-C-NH₂ ;
(N → C) Fethoc-C(1O2)TT-A(5)CC-A(5)GG(6)-CA(5)A-NH₂ ;
(N → C) Benzyl-C(1O2)TT-A(2O2)CC-A(5)GG(6)-CA(5)A-NH₂ ;
(N → C) Phényl-C(1O2)TT-A(5)CC-A(5)GG(6)-CA(5)A-NH₂ ;
(N → C) Fethoc-C(1O2)TT-A(5)CC-A(7)GG(5)-CA(5)A-NH₂ ;
(N → C) Fethoc-C(1O2)TT-A(5)CC-A(6)GG(2O2)-CA(5)A-NH₂ ;
(N → C) Fethoc-C(1O2)TT-A(5)CC-A(5)GG(6)-CA(5)A-Val-Lys-NH₂ ;
(N → C) Piv-C(1O2)TT-A(5)CC-A(5)GG(6)-CA(5)A-NH₂ ;
(N → C) Fmoc-Lys-Val-C(1O2)TT-A(5)CC-A(5)GG(6)-CA(5)A-NH₂ ;
(N → C) Ac-C(1O2)TT-A(5)CC-A(5)GG(6)-CA(5)A-NH₂ ;
(N → C) H-C(1O2)TT-A(5)CC-A(5)GG(6)-CA(5)A-Lys-NH₂ ;
(N → C) Piv-Arg-C(1O2)TT-A(5)CC-A(5)GG(6)-CA(5)A-Lys-NH₂ ;
(N → C) N-Phényl-N-méthyl-CTT-A(5)C(1O2)C-A(5)GG-C(1O2)AA(5)-G-Lys-NH₂ ;
(N → C) [N-(2-Phényléthyl)amino]carbonyl-CTT-A(5)C(1O2)C-A(4)G(5)G-C(1O2)AA(5)-G-NH₂ ;
(N → C) Benzoyl-Leu-C(1O2)TT-A(5)CC-A(5)GG(6)-CA(5)A-Lys-NH₂ ;
(N → C) Fethoc-C(1O3)TT-A(5)CC-A(5)GG(5)-CA(5)A-NH₂ ;
(N → C) Fethoc-C(1O2)TT-A(6)CC-A(6)GG(6)-CA(6)A-NH₂ ;
(N → C) Fethoc-TTT-TCC(1O2)-TTA(6)-CCA(6)-GG(6)C-A(6)A-NH₂ ;
(N → C) Fethoc-TTT-TCC(1O2)-TTA(6)-CC(1O3)A(6)-G-Lys-NH₂ ;
(N → C) Fethoc-TC(2O2)C-TTA(6)-CCA(6)-GG(6)C-A(6)A-NH₂ ;
(N → C) Me-Gly-TC(2O2)C-TTA(6)-CCA(6)-GG(6)C-A(6)A-NH₂ ;
(N → C) Fethoc-Lys-TTT-TCC(1O2)-TTA(6)-CCA(6)-GG(6)C-NH₂ ; et
(N → C) Fethoc-Arg-TCC(1O2)-TTA(5)-CCA(6)-GG(5)C-Lys-NH₂ ; dans lesquels
A, G, T et C sont des monomères PNA avec une nucléobase naturelle d'adénine, de guanine, de thymine et de cytosine, respectivement ;
C(pOq), A(p), A(pOq), G(p) et G(pOq) sont des monomères PNA avec une nucléobase non naturelle représentée par **la formule VI, la formule VII, la formule VIII, la formule IX,** et **la formule X,** respectivement ; 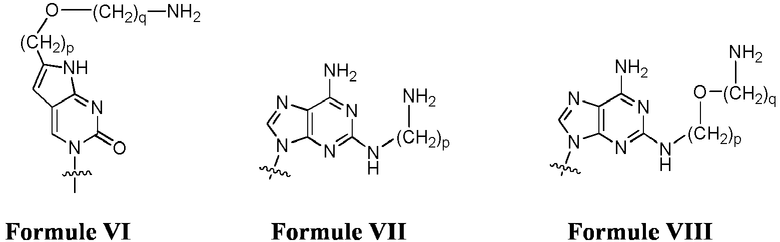 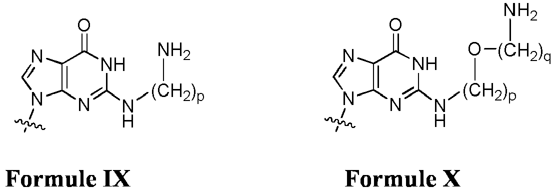 dans lesquelles,
p et q sont des entiers ; et
les abréviations pour les substituants des terminaisons N et C étant tels que spécifiquement décrits comme suit : « Fmoc- » est l'abréviation pour « [(9-fluorényl)méthyloxy]carbonyl- » ; « Fethoc- » pour « [2-(9-fluorényl)éthyl-1-oxy]carbonyl- » ; « Ac- » pour « acétyl- » ; « Benzoyl- » pour « benzènecabonyl- » ; « Piv- » pour « pivalyl- » ; « Méthyl- » pour « méthyl- » ; « n-Propyl- » pour « 1-(n-propyl)- » ; « H- » pour un groupe « hydrido- » ; « p-Toluènesulfonyl » pour « (4-méthylbenzène)-1-sulfonyl- » ; « -Lys- » pour un résidu d'acide aminé « lysine » ; « -Val- » pour un résidu d'acide aminé « valine » ; « -Leu- » pour un résidu d'acide aminé « leucine » ; « -Arg- » pour un résidu d'acide aminé « arginine » ; « -Gly- » pour un résidu d'acide aminé « glycine » ; « [N-(2-Phényléthyl)amino]carbonyl- » pour « [N-1-(2-phényléthyl)amino]carbonyl- » ; « Benzyl- » pour « 1-(phényl)méthyl- » ; « Phényl- » pour « phényl- » ; « Me- » pour « méthyl- » ; et « -NH₂ » pour un groupe « -amino » non substitué.

4. Dérivé d'acide nucléique peptidique selon la revendication 1, qui est choisi dans le groupe des composés fournis ci-après, ou sel pharmaceutiquement acceptable de celui-ci :
(N → C) Fmoc-GA(5)A-GC(1O2)C-A(5)GG-C(1O2)AA(5)-G-NH₂ ;
(N → C) Fethoc-GA(5)A-GC(1O2)C-A(5)GG-C(1O2)AA(5)-G-NH₂ ;
(N → C) Fmoc-GA(6)A-GC(1O2)C-A(6)GG-C(1O2)AA(6)-G-NH₂ ;
(N → C) Fmoc-G(6)AA(5)-GC(1O3)C-A(7)GG(5)-CA(5)A-G-NH₂ ;
(N → C) Fmoc-GA(5)A-GC(2O2)C-A(6)GG-C(1O5)AA(6)-G-NH₂ ;
(N → C) Fethoc-TG(6)C(1O2)-GGA(6)-AG(6)C-CA(6)G-GC(1O2)A-A(6)GG(6)-NH₂ ;
(N → C) Fethoc-C(1O2)TT-A(5)CC-A(5)GG-C(1O2)AA(5)-G-NH₂ ;
(N → C) Fethoc-C(1O2)TT-A(6)CC-A(5)GG-C(1O3)AA(5)-G-Val-Lys-NH₂ ;
(N → C) Ac-C(1O2)TT-A(5)CC-A(5)GG-C(1O2)TA(5)-G-NH₂ ;
(N → C) Piv-C(1O2)TT-A(5)CC-A(5)GG-C(1O2)AA(5)-G-NH₂ ;
(N → C) H-CTT-A(5)C(1O3)C-A(5)G(3)G-C(1O2)AA(5)-G-NH₂ ;
(N → C) n-Propyl-C(1O2)TT-A(5)CC-A(5)GG-C(1O2)AA(5)-G-NH₂ ;
(N → C) n-Propyl-CTT-A(5)C(2O2)C-A(3)G(2O3)G-C(1O2)AA(5)-G-NH₂ ;
(N → C) p-Toluènesulfonyl-CTT-A(5)C(1O2)C-A(8)G(5)G-C(1O2)AA(5)-G-NH₂ ;
(N → C) Benzoyl-CTT-A(5)C(1O5)C-A(5)G(2O2)G-C(1O2)AA(5)-G-NH₂ ;
(N → C) Fethoc-Lys-Leu-CTT-A(5)C(1O2)C-A(2O2)GG-C(1O2)AA(5)-G-Lys-NH₂ ;
(N → C) Fethoc-C(1O2)TT-A(5)CC-A(5)GG-C(1O5)AA(5)-G-NH₂ ;
(N → C) Fethoc-C(1O2)TT-A(5)CC-A(7)GG-C(1O2)AA(3)-G-NH₂ ;
(N → C) Fethoc-CTT-A(5)C(1O2)C-A(5)GT-C(1O2)TA(5)-G-NH₂ ;
(N → C) Fethoc-TC(1O2)C-TTA(6)-CCA(6)-GGC(1O2)-AA(6)G-G(6)-NH₂ ;
(N → C) Fethoc-TC(1O2)C-TTA(5)-CCA(5)-GGC(1O2)-AA(5)G-G(6)-NH₂ ;
(N → C) Fethoc-GA(5)T-AC(1O2)C-A(5)GG(6)-CAA(5)-G-NH₂ ;
(N → C) Fethoc-TA(5)C-CAG(6)-GC(1O2)A-A(5)GG(6)-C-NH₂ ;
(N → C) Fethoc-C(1O2)TT-A(5)CC-A(5)GG(6)-CA(5)A-NH₂ ;
(N → C) Fethoc-C(1O2)TT-A(5)CC-A(7)GG(5)-CA(5)A-NH₂ ;
(N → C) Fethoc-C(1O2)TT-A(5)CC-A(6)GG(2O2)-CA(5)A-NH₂ ;
(N → C) Piv-C(1O2)TT-A(5)CC-A(5)GG(6)-CA(5)A-NH₂ ;
(N → C) Ac-C(1O2)TT-A(5)CC-A(5)GG(6)-CA(5)A-NH₂ ;
(N → C) N-Phényl-N-méthyl-CTT-A(5)C(1O2)C-A(5)GG-C(1O2)AA(5)-G-Lys-NH₂ ;
(N → C) [N-(2-Phényléthyl)amino]carbonyl-CTT-A(5)C(1O2)C-A(4)G(5)G-C(1O2)AA(5)-G-NH₂ ;
(N → C) Fethoc-C(1O2)TT-A(6)CC-A(6)GG(6)-CA(6)A-NH₂ ;
(N → C) Fethoc-TTT-TCC(1O2)-TTA(6)-CCA(6)-GG(6)C-A(6)A-NH₂ ;
(N → C) Fethoc-TTT-TCC(1O2)-TTA(6)-CC(1O3)A(6)-G-Lys-NH₂ ; et
(N → C) Fethoc-TC(2O2)C-TTA(6)-CCA(6)-GG(6)C-A(6)A-NH₂.

5. Dérivé d'acide nucléique peptidique selon la revendication 1, choisi dans le groupe constitué par :
(N → C) Fethoc-C(1O2)TT-A(5)CC-A(5)GG-C(1O2)AA(5)-G-NH₂,
(N → C) Fethoc-TC(1O2)C-TTA(6)-CCA(6)-GGC(1O2)-AA(6)G-G(6)-NH₂,
(N → C) Fethoc- TC(1O2)C-TTA(5)-CCA(5)-GGC(1O2)-AA(5)G-G(6)-NH₂,
(N → C) Fethoc-TA(5)C-CAG(6)-GC(1O2)A-A(5)GG(6)-C-NH₂,
(N → C) Fethoc-C(1O2)TT-A(5)CC-A(5)GG(6)-CA(5)A-NH₂,
(N → C) Fethoc-C(1O2)TT-A(5)CC-A(6)GG(6)-CA(5)A-NH₂,
(N → C) Ac-C(1O2)TT-A(5)CC-A(5)GG(6)-CA(5)A-NH₂, ou
(N → C) Fethoc-C(1O2)TT-A(6)CC-A(6)GG(6)-CA(6)A-NH₂, ou sel pharmaceutiquement acceptable de celui-ci.

6. Dérivé d'acide nucléique peptidique selon l'une quelconque des revendications 1 à 5, destiné à être utilisé dans le traitement d'une indication dermatologique impliquant une activité androgène.

7. Dérivé d'acide nucléique peptidique selon l'une quelconque des revendications 1 à 5, destiné à être utilisé dans le traitement d'un état dermatologique impliquant une activité androgène.

8. Dérivé d'acide nucléique peptidique selon l'une quelconque des revendications 1 à 5, destiné à être utilisé dans le traitement de l'alopécie androgène.
